(19) Europäisches Patentamt / European Patent Office / Office européen des brevets

(11) **EP 3 561 703 B1**

(12) **EUROPÄISCHE PATENTSCHRIFT**

(45) Veröffentlichungstag und Bekanntmachung des Hinweises auf die Patenterteilung:
**20.01.2021 Patentblatt 2021/03**

(51) Int Cl.:
*C12N 15/10* (2006.01)     *C40B 40/10* (2006.01)
*G16B 20/00* (2019.01)     *G16B 40/00* (2019.01)

(21) Anmeldenummer: **18169334.2**

(22) Anmeldetag: **25.04.2018**

(54) **IDENTIFIZIEREN DER PAARUNG VON VARIABLEN DOMÄNEN AUS LEICHTEN UND SCHWEREN KETTEN VON ANTIKÖRPERN**

IDENTIFICATION OF THE MATING OF VARIABLE DOMAINS FROM LIGHT AND HEAVY CHAINS OF ANTIBODIES

IDENTIFICATION D'APPARIEMENT DE DOMAINES VARIABLES DES CHAÎNES LÉGÈRES ET LOURDES D'ANTICORPS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**30.10.2019 Patentblatt 2019/44**

(73) Patentinhaber: **Bayer AG**
**51373 Leverkusen (DE)**

(72) Erfinder:
- **STRERATH, Michael**
  **40477 Düsseldorf (DE)**
- **BENDER, Christian**
  **50823 Köln (DE)**
- **VÖLKER, Johanna**
  **14109 Berlin (DE)**
- **BOLIVAR LOPEZ, Julio Cesar**
  **40237 Düsseldorf (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**US-A1- 2013 178 370**

- **LINLING HE ET AL: "Hidden Lineage Complexity of Glycan-Dependent HIV-1 Broadly Neutralizing Antibodies Uncovered by Digital Panning and Native-Like gp140 Trimer", FRONTIERS IN IMMUNOLOGY, Bd. 8, 24. August 2017 (2017-08-24), XP55516159, DOI: 10.3389/fimmu.2017.01025**
- **REDDY SAI T ET AL: "Monoclonal antibodies isolated without screening by analyzing the variable-gene repertoire of plasma cells", NATURE BIOTECHNOLOGY, GALE GROUP INC, Bd. 28, Nr. 9, 1. September 2010 (2010-09-01), Seiten 965-969, 1, XP002693037, ISSN: 1087-0156, DOI: 10.1038/NBT.1673**

**Beschreibung**

Die vorliegende Erfindung befasst sich mit der Identifikation von Antikörpern und/oder Antikörperfragmenten aus einem Selektionsverfahren. Gegenstand der vorliegenden Erfindung sind ein Verfahren, ein System und ein Computerprogrammprodukt zur Identifikation von Paaren von Genen, die die variablen Domänen von leichten und schweren Ketten von selektierten Antikörpern und/oder Antikörperfragmenten kodieren.

Das humane Immunsystem bildet ein komplexes Netzwerk aus verschiedenen Organen, Zelltypen und Molekülen als Abwehrsystem gegen körperfremde Eindringlinge.

Antikörper, auch Immunglobuline genannt, sind Proteine aus der Klasse der Globuline, die in Wirbeltieren als Reaktion auf bestimmte Stoffe, so genannte Antigene, gebildet werden. Antikörper stehen im Dienste des Immunsystems; sie werden von einer Klasse weißer Blutzellen, den B-Lymphozyten, produziert.

Als Antigene wirken fast ausschließlich Makromoleküle oder an Partikel gebundene Moleküle, zum Beispiel Lipopolysaccharide an der Oberfläche von Bakterien. Ein bestimmtes Antigen induziert in der Regel die Bildung von nur wenigen bestimmten Antikörpern, die über spezifische, nicht-kovalente Bindungen zumeist nur diesen Fremdstoff erkennen.

Die spezifische Bindung von Antikörpern an die Antigene bildet einen wesentlichen Teil der Abwehr gegen die eingedrungenen Fremdstoffe.

Jeder Antikörper besteht aus zwei identischen schweren Ketten (engl.: *heavy chains,* H) und zwei identischen leichten Ketten (engl.: *light chains,* L), die durch kovalente Disulfidbrücken zu einer Ypsilon-förmigen Struktur miteinander verknüpft sind. Die leichten Ketten bestehen aus jeweils einer variablen und einer konstanten Domäne. Bezeichnet werden diese als $V_L$ und $C_L$. Die schweren Ketten hingegen haben jeweils eine variable und drei (IgG, IgA) bzw. vier (IgM, IgE) konstante Domänen. Bezeichnet werden diese analog als $V_H$ und $C_H1$, $C_H2$, $C_H3$.

Die variablen Domänen einer leichten und einer schweren Kette bilden die Antigenbindungsstelle; diese sind daher von besonderem Interesse für therapeutische, immunologische und/oder diagnostische Zwecke. Große Bibliotheken von Antikörpern und/oder Antikörperfragmenten werden erstellt und ausgewertet, um diese in der Medizin zu nutzen.

Eine weitverbreitete Technik zum Generieren und Charakterisieren von Antikörperbibliotheken ist die "Phage Display"-Methode, bei der das jeweilige Protein von Interesse als Fusionspolypeptid auf einem Bakteriophagen-Hüllprotein exprimiert und durch Bindung an immobilisierten oder löslichen biotinylierten Liganden (Antigenen) selektiert werden kann. Ein Phage, der auf diese Weise konstruiert wurde, kann als kompakte genetische Einheit betrachtet werden, welche sowohl die phänotypischen als auch die genotypischen Eigenschaften in sich vereint hat. Die "Phage Display"-Technologie ist erfolgreich auf Antikörper, Antikörperfragmente, Enzyme, DNA-Bindungsproteine usw. angewendet worden.

Um z.B. ein Phagen-Display von Antikörper-Bibliotheken zu verwenden, werden zunächst die relevanten Zellen aus dem Organismus isoliert. Dabei handelt es sich um Plasmazellen, die insbesondere in Blut, Knochenmark und Lymphknoten zu finden sind. Aus diesen Zellen wird mRNA isoliert, die dann in cDNA transkribiert wird.

Mit Hilfe der Polymerase-Kettenreaktion (PCR) werden aus der cDNA die Gene der variablen Domänen der leichten ($V_L$) und schweren Kette ($V_H$) der Antikörper vervielfältigt.

Jeder Gensatz wird mit dem verkürzten Gen des Hüllproteins pIII (minor coat protein) des M13-Phagen in einem speziellen Phagemid-Vektor ligiert und *Escherichia coli* damit transformiert.

Dadurch exprimieren die *E. coli* Bakterien pIII-Fusionsproteine mit scFv-Fragmenten oder Fab-Antikörperfragmenten. Die Fusionsproteine werden durch ein Signalpeptid ins Periplasma transportiert, dort falten sie sich zu einem funktionalen scFv bzw. durch Disulfidbrücke verbundenem Fab-Fragment. Die Fv- bzw. Fab-Anteile bleiben zunächst über das pIII-Fragment in der inneren E. coli-Membran verankert und binden beim Abschluss des Zusammenbaus des Phagen an das Kapsid.

Über das Hüllprotein pIII, das normalerweise für die Infektion der Bakterien verantwortlich ist, wird nach Koinfektion mit einem M13-Helferphagen das funktionale Antikörperfragment beim Reifungsprozess neugebildeter Phagen in deren Außenhülle eingebaut. Gleichzeitig wird der Phagemid mit der zugehörigen genetischen Information für das entsprechende Antikörperfragment ins Innere der neugebildeten Phagen eingebaut. Jeder dieser rekombinanten Phagen hat also theoretisch ein anderes Antikörperfragment auf seiner Oberfläche und gleichzeitig die zugehörigen Gene ($V_L$ und $V_H$) in seinem Inneren, vergleichbar mit den Milliarden von B-Zellen im (menschlichen) Körper.

In einem sogenannten Biopanning können die "bindenden" Phagen über die auf der Oberfläche exponierten Antikörperfragmente durch Wechselwirkung mit fixierten Liganden (Antigenen) aus dem milliardenfachen Hintergrund der irrelevanten Phagen selektiert werden.

Üblicherweise werden beim Biopanning mehrere Selektionszyklen (Panning-Runden) durchlaufen. Üblicherweise wird dazu eine Phage-Display Bibliothek einem Substrat ausgesetzt, so dass die Bindung von einigen Phagen stattfinden kann. Unspezifisch bindende und schwach bindende Phagen werden abgewaschen. Nach dem Waschen noch bindende und damit spezifische Phagen werden anschließend abgelöst (eluiert). Die eluierten Phagen werden vermehrt und in weiteren Panning-Runden erneut dem Substrat ausgesetzt, bis sich eine Population von gut bindenden Phagen anreichert.

Am Ende des Selektionsprozesses können aus den isolierten Phagen die zugehörigen Antikörpergene einfach isoliert und sequenziert werden. Die Sequenzierung ergibt dann Auskunft über die Baupläne der Antikörper(fragmente). Weitere Verfahren zur Selektion von Antikörpern und/oder Antikörperfragmenten sind in der Literatur beschrieben (siehe z.B. Sai T. Reddy et al.: Monoclonal antibodies isolated without screening by anaylzing the variable-gene repertoire of plasma cells, Nature Biotechnology Vol. 28 No. 9, Sept. 2010, 965-971).

Eine Standard-Methode zur Sequenzbestimmung ist die Didesoxymethode nach Sanger (Kettenabbruch-Synthese). Die Länge der so sequenzierten DNA-Abschnitte (Reads) kann mehr als 1000 Basenpaare erreichen, dies ist jedoch mit hohen Kosten und Zeitaufwand verbunden.

Die Diversität von Phage-Display-Bibliotheken liegt üblicherweise zwischen $10^7$ und $10^{11}$. Mit der Sequenzierungsmethode nach Sanger werden heute aber nur einige Hundert Antikörpergene sequenziert, d.h. nur von einem Bruchteil der $10^7$ bis $10^{11}$ Klone einer Phage-Display-Bibliothek wird die Identität bestimmt.

Eine Alternative stellt die Hochdurchsatz-Sequenzierung (engl.: *Next Generation Sequencing,* NGS) dar. Dank der Detektion von vielen parallel ablaufenden und räumlich getrennten Sequenzierungsreaktionen auf kleinster Fläche wird im Vergleich zum Standard schneller ein deutlich höherer Durchsatz bei kürzeren Read-Längen erreicht. NGS erreicht eine Größenordnung von $10^6$ Sequenzierungen. Damit ist es möglich, die Vielfalt und Qualität einer ganzen Bibliothek zu bestimmen.

Auch für den Selektionsprozess beim Durchlaufen mehrerer Biopanning-Zyklen bietet die NGS-Technologie einen großen Vorteil gegenüber der Standard-Sanger-Technologie. Die Sanger-Sequenzierung erlaubt nur die Bestimmung einiger weniger Antikörpergene, anhand derer üblicherweise nur die Selektionsrunde identifiziert wird, die für ein nachfolgendes Screening verwendet wird. Die Möglichkeit der NGS-Technologie, Sequenzinformationen von einer Vielzahl, wenn nicht sogar allen Klonen gewinnen zu können, würde einen tiefen Einblick in den Selektionsprozess geben und könnte einen nachfolgenden Screening-Schritt sogar erübrigen.

Die Herausforderung für Next Generation Sequenzierungssysteme liegt in der Bewältigung der Datenmengen, die (bio)informatisch ausgewertet werden müssen. Die Vielzahl von sequenzierten Genabschnitten muss richtig zusammengesetzt werden, um die Gene, die den gesuchten Antikörper kodieren, eindeutig bestimmen zu können.

Ein Problem - gerade bei einer großen Zahl von ermittelten Gensequenzen - stellt dabei die Identifizierung der richtigen $V_L$-$V_H$-Paare dar. Die variablen Domänen der leichten Kette ($V_L$) und der schweren Kette ($V_H$) eines Antikörpers werden durch unterschiedliche Gene kodiert. Werden bei einem Selektionsverfahren wie beispielsweise dem Biopanning viele Gensegmente sequenziert, fallen eine Vielzahl von Genen an, die die variablen Domänen der leichten und schweren Ketten kodieren. Unklar ist jedoch, welche Gene für leichte Ketten mit welchen Genen für schwere Ketten paarweise zu kombinieren sind, um die richtigen $V_L$-$V_H$-Paare und damit den gesuchten Antikörper zu bestimmen.

US 2013/178370 A1 beschreibt die Identifizierung von Antikörperfragmenten über Phagen-Display-Verfahren. Dabei wird *Next Generation Sequencing* zum Bestimmen der Gene verwendet. Paarungen von $V_L$- und $V_H$-Genen korrelieren mit der Reihenfolge der Häufigkeit der zugehörigen Proteine in einer Probe.

Linling et al. offenbaren ein Phage-Display Verfahren zur Identifizierung von scFv Antikörperfragmenten, die an HIV-1 Antigene binden. Vier Selektionszyklen werden durchgeführt, und nach jedem Zyklus werden die Plasmide aus den Bakterien extrahiert und mittels NGS sequenziert. Die bioinformatische Analyse beinhaltet das Bereitstellen von HC und LC Datensets. Nichtredundante scFv Klone werden identifiziert indem charakteristische, einzigartige HCDR3 - LCDR3 Paare zusammengestellt werden (Hidden Lineage Complexity of Glycan-Dependent HIV-1 Broadly Neutralizing Antibodies Uncovered by Digital Panning and Native-Like gp140 Trimer, Frontiers in Immunology, Bd. 8, 24. August 2017 (2017-08-24), XP55516159, DOI: 10.3389/fimmu.2017.01025).

Sai T. Reddy et al. berichten, VL-VH-Paare ließen sich anhand der Häufigkeiten der entsprechenden Gene auffinden (Monoclonal antibodies isolated without screening by anaylzing the variable-gene repertoire ofplasma cells, Nature Biotechnology Vol. 28 No. 9, Sept. 2010, 965-971).

In der Praxis hat sich jedoch gezeigt, dass eine Identifizierung von $V_L$-$V_H$-Paaren allein durch Zuordnung von Genen zueinander auf Basis der Häufigkeiten, mit denen sie auftreten, zu keinem befriedigenden Ergebnis führt.

Ausgehend vom beschriebenen Stand der Technik stellt sich damit die Aufgabe, aus einer Vielzahl von Genen für die variablen Domänen leichter und schwerer Ketten Paare von Genen zu identifizieren, die $V_L$-$V_H$-Paare von Antikörpern kodieren.

Erfindungsgemäß wird diese Aufgabe durch die Gegenstände der unabhängigen Ansprüche gelöst. Bevorzugte Ausführungsformen finden sich in den abhängigen Ansprüchen sowie in der vorliegenden Beschreibung.

Ein erster Gegenstand der vorliegenden Erfindung ist somit ein Verfahren umfassend die Schritte:

- Bereitstellen einer Bibliothek von Antikörpern und/oder Antikörperfragmenten
- Zuführen der Antikörper und/oder Antikörperfragmente einem Selektionsverfahren, wobei das Selektionsverfahren mindestens zwei Selektionszyklen, einen ersten Selektionszyklus und einen zweiten Selektionszyklus, umfasst

- Sequenzieren der Gene der Antikörper und/oder Antikörperfragmente nach dem ersten Selektionszyklus und nach dem zweiten Selektionszyklus und Ermitteln von $V_L$-Genen, die die variablen Domänen von leichten Ketten der Antikörper und/oder Antikörperfragmente kodieren und von $V_H$-Genen, die die variablen Domänen der schweren Ketten der Antikörper und/oder Antikörperfragmente kodieren

- Ermitteln von Merkmalen zu den $V_L$-Genen und $V_H$-Genen aus dem ersten Selektionsverfahren und aus dem zweiten Selektionsverfahren, wobei die Merkmale zu den $V_L$-Genen und $V_H$-Genen die folgenden Merkmale umfassen:

  • eindeutige Kennungen der ermittelten $V_H$-Gene,

  • eindeutige Kennungen der ermittelten $V_L$-Gene,

  • Häufigkeiten der $V_H$-Gene in dem Pool aus dem zweiten Selektionszyklus,

  • Häufigkeiten der $V_L$-Gene in dem Pool aus dem zweiten Selektionszyklus,

  • die Häufigkeiten der $V_H$-Gene in dem Pool aus dem ersten Selektionszyklus, und

  • die Häufigkeiten der $V_L$-Gene in dem Pool aus dem ersten Selektionszyklus

- Bilden von Merkmalsvektoren für Paare von $V_L$-Genen und $V_H$-Genen aus dem zweiten Selektionszyklus auf Basis der ermittelten Merkmale aus dem ersten Selektionszyklus und dem zweiten Selektionszyklus

- Zuführen der Merkmalsvektoren einem Klassifikationsmodell, das für die jeweiligen Paare eine Klassifizierung in eine von mindestens zwei Klassen vornimmt, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören

- Ausgeben von Informationen zumindest zu Paaren in der ersten Klasse.

Ein weiterer Gegenstand der vorliegenden Erfindung ist ein System umfassend:

- eine Eingabeeinheit,
- eine Steuer- und Recheneinheit,
- eine Merkmalsvektorerzeugungseinheit,
- eine Klassifikationseinheit, und
- eine Ausgabeeinheit,

  - wobei die Steuer- und Recheneinheit konfiguriert ist, Merkmale zu $V_L$-Genen und $V_H$-Genen, die variable Domänen von leichten und schweren Ketten von Antiköpern und/oder Antikörperfragmenten kodieren, über die Eingabeeinheit zu erfassen,

    o wobei die Antikörper und/oder Antikörperfragmente aus einem Selektionsverfahren umfassend mindestens zwei Selektionszyklen, einen ersten Selektionszyklus und einen zweiten Selektionszyklus, stammen,
    o wobei die Merkmale zu den $V_L$-Genen und $V_H$-Genen die folgenden Merkmale umfassen:

      • eindeutige Kennungen der ermittelten $V_H$-Gene,

      • eindeutige Kennungen der ermittelten $V_L$-Gene,

      • Häufigkeiten der $V_H$-Gene in dem Pool aus dem zweiten Selektionszyklus,

      • Häufigkeiten der $V_L$-Gene in dem Pool aus dem zweiten Selektionszyklus,

      • die Häufigkeiten der $V_H$-Gene in dem Pool aus dem ersten Selektionszyklus, und

      • die Häufigkeiten der $V_L$-Gene in dem Pool aus dem ersten Selektionszyklus,

  - wobei die Steuer- und Recheneinheit konfiguriert ist, die Merkmalsvektorerzeugungseinheit zu veranlassen, auf Basis der erfassten Merkmale Merkmalsvektoren für Paare von $V_L$- und $V_H$- Genen aus dem zweiten Selektionszyklus zu bilden,

- wobei die Steuer- und Recheneinheit konfiguriert ist, die Klassifikationseinheit zu veranlassen, die Paare von $V_L$- und $V_H$-Genen anhand ihrer Merkmalsvektoren einer von mindestens zwei Klassen zuzuordnen, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören,

- wobei die Steuer- und Recheneinheit konfiguriert ist, die Ausgabeeinheit zu veranlassen, zumindest Informationen über Paare auszugeben, die der ersten Klasse zugeordnet sind.

Ein weiterer Gegenstand der vorliegenden Erfindung ist ein Computerprogrammprodukt umfassend einen Datenträger, und Programmcode, der auf dem Datenträger gespeichert ist, und der einen Computer, in dessen Arbeitsspeicher der Programmcode geladen ist, dazu veranlasst, die folgenden Schritte auszuführen:

- Erfassen von Merkmalen zu $V_L$-Genen und $V_H$-Genen, die variable Domänen von leichten und schweren Ketten von Antiköpern und/oder Antikörperfragmenten kodieren,

  ∘ wobei die Antikörper und/oder Antikörperfragmente aus einem Selektionsverfahren umfassend mindestens zwei Selektionszyklen, einen ersten Selektionszyklus und einen zweiten Selektionszyklus, stammen,

  ∘ wobei die Merkmale zu den $V_L$-Genen und $V_H$-Genen die folgenden Merkmale umfassen:

  • eindeutige Kennungen der ermittelten $V_H$-Gene,

  • eindeutige Kennungen der ermittelten $V_L$-Gene,

  • Häufigkeiten der $V_H$-Gene in dem Pool aus dem zweiten Selektionszyklus,

  • Häufigkeiten der $V_L$-Gene in dem Pool aus dem zweiten Selektionszyklus,

  • die Häufigkeiten der $V_H$-Gene in dem Pool aus dem ersten Selektionszyklus, und

  • die Häufigkeiten der $V_L$-Gene in dem Pool aus dem ersten Selektionszyklus,

  - Bilden von Merkmalsvektoren für Paare von $V_L$- und $V_H$-Genen aus dem zweiten Selektionszyklus auf Basis der erfassten Merkmale,

  - Zuordnen der Paare von $V_L$- und $V_H$-Genen auf Basis ihrer Merkmalsvektoren zu einer von mindestens zwei Klassen, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören,

  - Ausgeben von Informationen zumindest über Paare, die der ersten Klasse zugeordnet sind.

Die Erfindung wird nachstehend näher erläutert, ohne zwischen den Erfindungsgegenständen zu unterscheiden. Die nachfolgenden Erläuterungen sollen vielmehr für alle Erfindungsgegenstände in analoger Weise gelten, unabhängig davon, in welchem Kontext sie erfolgen.
Wenn in der vorliegenden Beschreibung oder in den Patentansprüchen Schritte in einer Reihenfolge genannt werden, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden können; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen dar.
Ein Ausgangspunkt für die vorliegende Erfindung ist eine Bibliothek von Antikörpern und/oder Antikörperfragmenten. Diese Antikörper und/oder Antikörperfragmente werden einem Selektionsverfahren zugeführt, um Antikörper und/oder Antikörperfragmente auf Basis ihrer phänotypischen Eigenschaften zu selektieren. Bei dem Selektionsverfahren kann es sich beispielsweise um ein Biopanning-Verfahren handeln. Es kann sich auch um ein Immonisierungsverfahren handeln, wie es zum Beispiel in Nature Biotechnology Vol. 28 No. 9, Sept. 2010, 965-971 beschrieben ist. Weitere Selektionsverfahren sind denkbar.

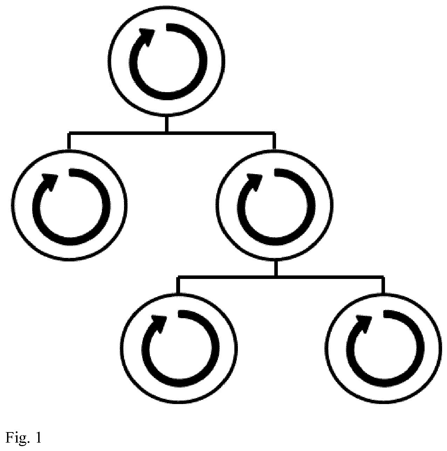

Ein Selektionsverfahren im Sinn der vorliegenden Erfindung umfasst mehrere Selektionszyklen. Es sind mindestens zwei Selektionszyklen vorhanden, ein erster Selektionszyklus und ein zweiter Selektionszyklus. Die Selektionszyklen können in einer Selektionshierarchie nebeneinander und/oder übereinander angeordnet sein. Figur 1 zeigt ein Beispiel für eine Selektionshierarchie mit drei Ebenen. In der ersten Ebene wird eine Bibliothek von Antikörpern und/oder Antikörperfragmenten einem ersten Selektionszyklus zugeführt. Das Ergebnis des ersten Selektionszyklus ist ein Pool selektierter Antikörper und/oder Antikörperfragmente. Die selektierten Antikörper und/oder Antikörperfragmente werden in der zweiten Ebene zwei Selektionszyklen zugeführt (links und rechts). In der Regel handelt es sich bei den Selektionszyklen in der zweiten Ebene um verschiedene Selektionszyklen. Die Ergebnisse der Selektionen in der zweiten Ebene sind wiederum zwei Pools von selektierten Antikörpern und/oder Antikörperfragmenten. Der aus dem rechten Ast der zweiten Ebene resultierende Pool an Antikörpern und/oder Antikörperfragmenten wird in der dritten Ebene erneut zwei Selektionszyklen ausgesetzt, die wiederum zwei Pools zum Ergebnis haben. In der Regel werden die Antikörper und/oder Antikörperfragmente beim Durchlaufen einer Selektionshierarchie von oben nach unten (z.B. von der ersten Ebene über die zweite Ebene zur dritten Ebene) einem zunehmenden Selektionsdruck ausgesetzt. Durchläuft man eine Selektionshierarchie von oben nach unten (z.B. von der ersten Ebene über die zweite Ebene zur dritten Ebene), nimmt die Diversität der Antikörper und/oder Antikörperfragmente in den jeweiligen Pools ab.

In einer bevorzugten Ausführungsform sind der erste Selektionszyklus und der zweite Selektionszyklus in der Selektionshierarchie des Selektionsverfahrens unmittelbar übereinander angeordnet. Vorzugsweise ist der zweite Selektionszyklus dem ersten Selektionszyklus unmittelbar nachfolgend angeordnet, d.h. die aus dem ersten Selektionszyklus stammenden (selektierten) Antikörper und/oder Antikörperfragmente werden (ggf. nach einer Vermehrung und weiteren üblichen Schritten in einem Sequenzierungsverfahren) dem zweiten Selektionszyklus zugeführt.

Es ist aber auch denkbar, dass der erste Selektionszyklus und der zweite Selektionszyklus in der Selektionshierarchie des Selektionsverfahrens nebeneinander angeordnet sind.

In einem nachfolgenden Schritt werden Gene der selektierten Antikörper und/oder Antikörperfragmente sequenziert.

Ziel der Sequenzierung ist die Ermittlung von $V_L$-Genen, die die variablen Domänen von leichten Ketten der Antikörper und/oder Antikörperfragmente kodieren und von $V_H$-Genen, die die variablen Domänen der schweren Ketten der Antikörper und/oder Antikörperfragmente kodieren.

Es ist denkbar, dass ein solcher Sequenzierungsschritt nach jedem Selektionszyklus durchgeführt wird. Denkbar ist auch, dass ein solcher Sequenzierungsschritt erst nach Durchlaufen einer Hierarchie von mehreren Zyklen durchgeführt wird. Es ist denkbar, dass zunächst eine gewisse Anreicherung spezifisch bindender Antikörper und/oder Antikörperfragmente erreicht werden soll, bevor eine Charakterisierung durch Sequenzierung stattfindet. Es werden jedoch zumindest Gene der Antikörper und/oder Antikörperfragmente in dem Pool aus dem ersten Selektionszyklus und in dem Pool aus dem zweiten Selektionszyklus sequenziert.

In einem weiteren Schritt des erfindungsgemäßen Verfahrens werden Merkmale zu den $V_L$- und $V_H$-Genen erfasst.

Erfindungsgemäß werden mindestens Merkmale zu den $V_L$- und $V_H$-Genen aus dem ersten Selektionszyklus und aus dem zweiten Selektionszyklus erfasst, da diese Merkmale bei der Bildung von Merkmalsvektoren zur Identifizierung von $V_L$-$V_H$-Paaren miteinander kombiniert werden. Es ist denkbar, Merkmale zu den $V_L$- und $V_H$-Genen aus weiteren Selektionszyklen zu erfassen.

Wichtige Merkmale zu $V_L$- und $V_H$-Genen sind ihre Häufigkeiten, mit denen sie auftreten. Die Häufigkeiten werden vorzugsweise per Next-Generation-Sequencing-Verfahren erfasst, indem die fragmentierte genetische Information von $V_H$ und $V_L$ Sequenzen mit Hilfe gepaarter Erkennungssequenzen (Primer-Paare) zu vollständigen $V_H$ und $V_L$ Ketten (oder Teilen davon) assembliert werden. Die eindeutigen Sequenzen werden dann gezählt und in Form von DNA-Counts ausgegeben.

Im Unterschied zu dem im Stand der Technik beschriebenen Verfahren werden erfindungsgemäß Merkmale (vorzugsweise Häufigkeiten) aus mehreren Selektionszyklen erfasst und zur Ermittlung von $V_L$-$V_H$-Paaren verwendet.

Neben den Häufigkeiten können weitere Merkmale zu den $V_L$- und $V_H$-Genen erfasst werden. Dazu zählen beispielsweise auch Informationen über die in den Selektionszyklen verwendeten Substrate, Parameter zur Durchführung der Sequenzierungszyklen (Konzentrationen, Temperaturen, Medien u.a.), Struktur der Sequenzierungshierarchie (Anzahl der Ebenen und/oder Verzweigungen) u.a.

Die erfassten Merkmale (wie beispielsweise die Häufigkeiten der $V_L$- und $V_H$-Gene) fließen in einem nachfolgenden Schritt in die Erzeugung von Merkmalsvektoren ein. Ein Merkmalsvektor (engl.: *feature vector*) fasst die (vorzugsweise numerisch) parametrisierbaren Eigenschaften (Merkmale) eines Objekts in vektorieller Weise zusammen. Verschiedene, für das Objekt charakteristische Merkmale bilden die verschiedenen Dimensionen dieses Vektors. Die Gesamtheit der möglichen Merkmalsvektoren nennt man den Merkmalsraum. Merkmalsvektoren erleichtern eine automatische Klassifikation, da sie die zu klassifizierenden Eigenschaften stark reduzieren.

Das Objekt ist im vorliegenden Fall ein Paar von $V_L$-Genen und $V_H$-Genen. Ein Merkmalsvektor lässt sich für jedes Paar von $V_L$-Genen und $V_H$-Genen erzeugen. Der Merkmalsvektor charakterisiert das jeweilige Paar.

Folgende Merkmale können in den Merkmalsvektor für ein Paar von $V_L$-Genen und $V_H$-Genen aufgenommen werden:

- Information über das betrachtete $V_H$-Gen (z.B. eine eindeutige Kennung)

- Information über das betrachtete $V_L$-Gen (z.B. eine eindeutige Kennung)

- die absolute Zahl (Häufigkeit) des $V_H$-Gens in dem Pool aus dem zweiten Selektionszyklus: $A(V_H)$

- die absolute Zahl (Häufigkeit) des betrachteten $V_L$-Gens in dem Pool aus dem zweiten Selektionszyklus: $A(V_L)$

- die absolute Zahl (Häufigkeit) unterschiedlicher $V_H$-Gene in dem Pool aus dem zweiten Selektionszyklus: $numV_H$

- die absolute Zahl (Häufigkeit) unterschiedlicher $V_L$-Gene in dem Pool aus dem zweiten Selektionszyklus: $numV_L$

- die absolute Zahl (Häufigkeit) desjenigen $V_H$-Gens, das in dem Pool aus dem zweiten Selektionszyklus am häufigsten vorhanden ist: $maxV_H$

- die absolute Zahl (Häufigkeit) desjenigen $V_L$-Gens, das in dem Pool aus dem zweiten Selektionszyklus am häufigsten vorhanden ist: $maxV_L$

- die relative Häufigkeit des $V_H$-Gens in dem Pool aus dem zweiten Selektionszyklus (bezogen auf die Zahl des am häufigsten in dem Pool aus dem zweiten Selektionszyklus auftretenden $V_H$-Gens): $relV_H = A(V_H)/maxV_H$

- die relative Häufigkeit des $V_L$-Gens in dem Pool aus dem zweiten Selektionszyklus (bezogen auf die Zahl des am häufigsten in dem Pool aus dem zweiten Selektionszyklus auftretenden $V_L$-Gens): $relV_L = A(V_L)/maxV_L$

- der Unterschied (Abstand) zwischen der Häufigkeit des betrachteten $V_H$-Gens in dem Pool aus dem zweiten Selektionszyklus und der Häufigkeit des betrachteten $V_L$-Gens in dem Pool aus dem zweiten Selektionszyklus (als Betrag):: $diff = |A(V_H) - A(V_L)|$

- der relative Unterschied (relativer Abstand) zwischen der Häufigkeit des $V_H$-Gens in dem Pool aus dem zweiten Selektionszyklus und der Häufigkeit des $V_L$-Gens in dem Pool aus dem zweiten Selektionszyklus (in Bezug auf die Häufigkeit desjenigen Gens in dem Pool aus dem zweiten Selektionszyklus, das häufiger auftritt): $reldiff = |A(V_H) - A(V_L)| / Max(A(V_H), A(V_L))$, wobei $Max(A(V_H), A(V_L)) = A(V_H)$ für $A(V_H) > A(V_L)$ und $Max(A(V_H), A(V_L)) = A(V_L)$ für $A(V_L) \geq A(V_H)$

- die Zahl der Selektionszyklen (Ebenen), die vor dem zweiten Selektionszyklus durchlaufen worden sind: $prevnum$

- die absolute Zahl des $V_H$-Gens in dem Pool aus dem ersten Selektionszyklus: $prevA(V_H)$

- die absolute Zahl des $V_L$-Gens in dem Pool aus dem ersten Selektionszyklus: $prevA(V_L)$

- der Unterschied (Abstand) zwischen der Häufigkeit des $V_H$-Gens aus dem Pool des ersten Selektionszyklus und der Häufigkeit des $V_L$-Gens aus dem Pool des ersten Selektionszyklus (als Betrag): $prevdiff = |prevA(V_H) - prevA(V_L)|$

- die relative Veränderung der Zahl der $V_H$-Gene vom ersten Selektionszyklus zum zweiten Selektionszyklus: $prevRelDiffV_H = (|A(V_H) - prevA(V_H)|) / Max((A(V_H), prevA(V_H)))$, wobei $Max((A(V_H), prevA(V_H)) = A(V_H)$ für $A(V_H) > prevA(V_H)$ und $Max((A(V_H), prevA(V_H)) = prevA(V_H)$ für $prevA(V_H) \geq A(V_H)$

- die relative Veränderung der Zahl der $V_L$-Gene vom ersten Selektionszyklus zum zweiten Selektionszyklus: $prevRelDiffV_L = (|A(V_H) - prevA(V_L)|) / Max((A(V_L), prevA(V_L)))$, wobei $Max((A(V_L), prevA(V_L)) = A(V_L)$ für $A(V_L) > prevA(V_L)$ und $Max((A(V_L), prevA(V_L)) = prevA(V_L)$ für $prevA(V_L) \geq A(V_L)$

Es ist denkbar, dass neben den genannten Merkmalen weitere Informationen in die Erzeugung von Merkmalsvektoren einfließen. Beispielsweise können Häufigkeiten von $V_H$-Genen und $V_L$-Genen in Pools aus weiteren Selektionszyklen als Merkmale erfasst werden und in die Erzeugung von Merkmalsvektoren einfließen. Wie oben beschrieben können auch Merkmale zur Selektionshierarchie und/oder Parameter der einzelnen Selektionszyklen erfasst werden und in die Erzeugung von Merkmalsvektoren einfließen.

Die Merkmalsvektoren werden einem Klassifikationsmodell zugeführt. Das Klassifikationsmodell ordnet jedes Paar von $V_L$- und $V_H$-Genen auf Basis des jeweiligen Merkmalvektors einer von mindestens zwei Klassen zu. Eine erste Klasse umfasst diejenigen Paare, die variable Domänen der leichten und schweren Ketten kodieren, die zum selben Antikörper und/oder Antikörperfragment gehören. Eine zweite Klasse umfasst diejenigen Paare, die variable Domänen der leichten und schweren Ketten kodieren, die nicht zum selben Antikörper und/oder Antikörperfragment gehören.

Vereinfacht ausgedrückt, gibt das Klassifikationsmodell eine Auskunft darüber, ob ein $V_L$-Gen und ein $V_H$-Gen zusammengehören oder nicht. Sie gehören dann zusammen, wenn das $V_L$-Gen die variable Domäne einer leichten Kette eines Antikörpers und/oder Antikörperfragments kodiert, und das $V_H$-Gen die variable Domäne einer schweren Kette desselben Antikörpers und/oder desselben Antikörperfragments kodiert. In einem solchen Fall wird das Paar von $V_L$- und $V_H$-Genen auch als (richtiges) $V_L$-$V_H$-Paar bezeichnet. Sie gehören dann nicht zusammen, wenn das $V_L$-Gen die variable Domäne einer leichten Kette eines Antikörpers und/oder Antikörperfragments kodiert, und das $V_H$-Gen die variable Domäne einer schweren Kette eines anderen Antikörpers und/oder eines anderen Antikörperfragments kodiert.

Es ist denkbar, dass mehr als zwei Klassen vorhanden sind. Denkbar sind beispielsweise drei Klassen: eine erste Klasse, die Paare umfasst, bei denen die Wahrscheinlichkeit sehr groß ist, dass sie zusammengehören, eine zweite Klassen, die Paare umfasst, bei denen die Wahrscheinlichkeit sehr gering ist, dass sie zusammengehören, und eine dritte Klasse, die Paare umfasst, die weder der ersten noch der zweiten Klasse zuordenbar sind. Für die Paare der dritten Klasse herrscht somit eine gewisse Unsicherheit, ob es sich um $V_L$-$V_H$-Paare handelt oder nicht. Die jeweiligen Wahrscheinlichkeiten lassen sich aus dem Lernprozess, mit dem das jeweilige Klassifikationsmodell erstellt worden ist, ermitteln.

Das Klassifikationsmodell wird vorzugsweise auf Basis von selbstlernenden Algorithmen erstellt. Besonders bevorzugt wird das Klassifikationsmodell mittels überwachtem Lernen erstellt (überwachte Klassifikation).

Die Erstellung des Klassifikationsmodells kann beispielsweise mit bekannten Antikörpern und/oder Antikörperfragmenten durchgeführt werden. Für diese sind auch die $V_L$-$V_H$-Paare bekannt. Die bekannten Antikörper und/oder Antikörperfragmente können beispielsweise in Form einer Phage-Display Bibliothek einem Biopanning unterzogen werden, wobei nach allen oder einigen Zyklen die Gensequenzen bestimmt und die Häufigkeiten der Gene ermittelt werden. Mit diesen Daten wird ein Klassifikationssystem trainiert.

Für die Erstellung von Klassifikationsmodellen gibt es eine Vielzahl an Methoden, wie beispielsweise Random Forest oder Gradient Boosting.

Das Ergebnis ist ein Klassifikationsmodell, das auch auf unbekannte Antikörper und/oder Antikörperfragmente anwendbar ist. Die Zuordnung von unbekannten Paaren von $V_L$- und $V_H$-Genen einer der Klassen des Klassifikationsmodell ist umso genauer, je ähnlicher das Selektionsverfahren mit den unbekannten Antikörpern und/oder Antikörperfragmenten gegenüber dem Selektionsverfahren mit den bekannten Antikörpern und/oder Antikörperfragmenten ist. Zum Beispiel ist die Genauigkeit höher, wenn die gleichen Substrate (Antigene) verwendet werden, und niedriger, wenn unterschiedliche Substrate verwendet werden. Es hat sich jedoch herausgestellt, dass die Übertragbarkeit für eine Vielzahl an Selektionsverfahren und Antikörpern und/oder Antikörperfragmenten gegeben ist.

Für ein beliebiges Paar von $V_L$- und $V_H$-Genen kann also auf Basis des Klassifikationsmodells eine Aussage getroffen werden, ob sie (mit einer definierten Wahrscheinlichkeit) zusammengehören oder nicht. Diese Information kann in einem nächsten Schritt ausgegeben werden. Die Ausgabe kann beispielsweise auf einem Bildschirm eines Computers erfolgen. Die Information kann auch über einen Drucker ausgedruckt oder in einem Datenspeicher gespeichert werden.

In einer bevorzugten Ausführungsform ist das Selektionsverfahren ein Biopanning-Verfahren und als Merkmale für die Erzeugung von Merkmalsvektoren werden Häufigkeiten der $V_L$- und $V_H$-Gene in den Pools aus mindestens zwei Selektionszyklen verwendet.

Ein Ausgangspunkt für ein Biopanning-Selektionsverfahren ist eine erste Phage-Display Bibliothek. Die Bakteriophagen (kurz: Phagen) tragen Antikörperfragmente auf deren Außenhüllen; gleichzeitig tragen sie die zugehörigen Gene, die die Antikörperfragmente kodieren, in ihrem Inneren.

Es gilt, diejenigen Antikörper und/oder Antikörper-Fragmente, die eine Antigen-Antikörper-Reaktion mit einem oder mehreren definierten Antigenen eingehen, zu identifizieren und zu selektieren. Insbesondere gilt es, diejenigen Antikörper und/oder Antikörper-Fragmente zu identifizieren und zu selektieren, die gegenüber einem oder mehreren Epitopen eines oder mehrerer Antigene eine vergleichsweise hohe Affinität aufweisen. Diese hohe Affinität zeigt sich in einem besonders stabilen Antigen-Antikörper-Komplex mit einer hohen Komplexbildungskonstanten.

Denkbar ist auch, dass Antikörper und/oder Antikörper-Fragmente zu identifizieren und zu selektieren sind, die selektiv an verschiedene Epitope eines oder mehrerer Antigene binden.

Ein Biopanning-Verfahren (kurz: Biopanning) umfasst üblicherweise mehrere Zyklen (Selektionszyklen).

Ein Biopanning-Zyklus umfasst mindestens die folgenden Schritte:

(1) Bereitstellen einer Bibliothek von Phagen, bei denen Antikörper und/oder Antikörperfragmente als Fusionspolypeptide auf Phagen-Hüllproteinen exprimiert sind,
(2) Inkubieren der Phagen mit einem Substrat,
(3) Separieren der an das Substrat bindenden Phagen von den nicht-bindenden Phagen.

Üblicherweise werden die bindenden Phagen im Anschluss an Schritt (3) vermehrt. Das Ergebnis ist eine neue (zweite) Phage-Display Bibliothek, die wiederum einem Substrat ausgesetzt wird, um die an das Substrat bindenden von den nicht-bindenden Phagen zu trennen, und so fort. Denkbar ist aber auch, dass die nicht-bindenden Phagen im Anschluss an Schritt (3) vermehrt und einem weiteren Zyklus zugeführt werden.

Es ist denkbar, dass sich die selektierten Phagen im Hinblick auf ihr Vermehrungsverhalten unterscheiden. Es ist denkbar, dass Unterschiede im Vermehrungsverhalten zu einem Anreicherungseffekt in Bezug auf Phagen, die sich besonders gut vermehren lassen, führt.

Ein Substrat weist Antigene und/oder Antigenfragmente auf, die mit den exprimierten Antikörperfragmenten der Phagen in Wechselwirkung treten, d.h. stabile Antigen-Antikörper-Komplexe bilden und damit die entsprechenden Phagen binden können.

Es ist denkbar, dass bei jedem Zyklus das gleiche Substrat mit den gleichen Antigenen und/oder Antigenfragmenten verwendet werden. In einem solchen Fall kommt es im Verlauf der Zyklen zu einer Anreicherung von stark mit den Antigenen und/oder Antigenfragmenten wechselwirkenden Antikörpern und/oder Antikörperfragmenten.

Es ist aber auch denkbar, dass in einzelnen Zyklen verschiedene Substrate mit verschiedenen Antigenen und/oder Antigenfragmenten verwendet werden. Ein Grund dafür kann sein, dass Antikörper und/oder Antikörperfragmente selektiert werden sollen, die eine Affinität gegenüber mehreren Antigenen und/oder Antigenfragmenten aufweisen.

Denkbar ist weiterhin, dass in einem Zyklus Antikörper und/oder Antikörperfragmente selektiert werden, die möglichst wenig an ein Antigen und/oder Antigenfragment binden.

Am Ende eines Zyklus können die Gene der Antikörper und/oder Antikörperfragmente auf den bindenden Phagen sequenziert und die Häufigkeiten der Gene ermittelt werden.

Erfindungsgemäß werden mindestens zwei Selektionszyklen durchlaufen. Sind die Selektionszyklen in der Selektionshierarchie übereinander angeordnet, werden mindestens die folgenden Schritte durchlaufen:

(1) Bereitstellen einer Bibliothek von Phagen, bei denen Antikörper und/oder Antikörperfragmente als Fusionspolypeptide auf Phagen-Hüllproteinen exprimiert sind,
(2) Inkubieren der Phagen mit einem Substrat,
(3) Separieren der an das Substrat bindenden Phagen von den nicht-bindenden Phagen,
(4) Sequenzieren der Gene der Antikörper und/oder Antikörperfragmente auf den bindenden oder nicht-bindenden Phagen und Ermitteln von $V_L$-Genen, die die variablen Domänen von leichten Ketten der Antikörper und/oder Antikörperfragmente kodieren und von $V_H$-Genen, die die variablen Domänen der schweren Ketten der Antikörper und/oder Antikörperfragmente kodieren
(5) Ermitteln der Häufigkeiten der $V_L$-Gene und der $V_H$-Gene,
(6) Vermehren der bindenden oder nicht-bindenden Phagen und dabei Bilden einer neuen Bibliothek von Phagen,

(1') Bereitstellen der neuen Bibliothek von Phagen,
(2') Inkubieren der Phagen mit einem Substrat,
(3') Separieren der an das Substrat bindenden Phagen von den nicht-bindenden Phagen,
(4') Sequenzieren der Gene der Antikörper und/oder Antikörperfragmente auf den bindenden oder nicht-bindenden Phagen und Ermitteln von $V_L$-Genen, die die variablen Domänen von leichten Ketten der Antikörper und/oder Antikörperfragmente kodieren und von $V_H$-Genen, die die variablen Domänen der schweren Ketten der Antikörper und/oder Antikörperfragmente kodieren
(5') Ermitteln der Häufigkeiten der $V_L$-Gene und der $V_H$-Gene.

Als Ergebnisse nach Durchlaufen der Schritte (1) bis (5') liegen vor:

- selektierte Antikörper und/oder Antikörperfragmente

- Sequenzen der $V_L$-Gene und der $V_H$-Gene, die die variablen Domänen von leichten und schweren Ketten der selektierten Antikörper und/oder Antikörperfragmente kodieren

- die Häufigkeit der $V_L$-Gene und der $V_H$-Gene nach Durchlaufen des Zyklus (1')→(2') →(3') sowie der Häufigkeiten der $V_L$-Gene und der $V_H$-Gene nach Durchlaufen des vorangegangenen Zyklus (1)→(2) →(3).

Die in den Schritten (5) und (5') ermittelten Häufigkeiten (und ggf. weitere Merkmale) können in die Bildung von Merkmalsvektoren einfließen. Mittels eines Klassifikationsmodels lässt sich für jedes Paar von $V_L$- und $V_H$-Genen ermitteln, ob sie zusammengehören oder nicht.

Eine bevorzugte Ausführungsform des erfindungsgemäßen Verfahrens umfasst somit die folgenden Schritte:

(1) Bereitstellen einer Bibliothek von Phagen, bei denen Antikörper und/oder Antikörperfragmente als Fusionspolypeptide auf Phagen-Hüllproteinen exprimiert sind,

(2) Inkubieren der Phagen mit einem Substrat,

(3) Separieren der an das Substrat bindenden Phagen von den nicht-bindenden Phagen,

(4) Sequenzieren der Gene der Antikörper und/oder Antikörperfragmente auf den bindenden oder nicht-bindenden Phagen und Ermitteln von $V_L$-Genen, die die variablen Domänen von leichten Ketten der Antikörper und/oder Antikörperfragmente kodieren und von $V_H$-Genen, die die variablen Domänen der schweren Ketten der Antikörper und/oder Antikörperfragmente kodieren

(5) Ermitteln der Häufigkeiten der $V_L$-Gene und der $V_H$-Gene,

(6) Vermehren der bindenden oder nicht bindenden Phagen und dabei Bilden einer neuen Bibliothek von Phagen und *n*-faches Wiederholen der Schritte (1) bis (5), wobei in Schritt (1) die jeweils neue Bibliothek von Phagen eingesetzt wird, wobei n eine ganze Zahl und größer als 0 ist, und wobei eine Durchführung der Schritte (1) bis (4) einen Biopanning-Zyklus darstellt,

(7) Bilden von Merkmalsvektoren für Paare von $V_L$-Genen und $V_H$-Genen, wobei als Merkmale zumindest die Häufigkeiten der $V_L$-Gene und der $V_H$-Gene des aktuellen Biopanning-Zyklus und eines vorangegangenen Biopanning-Zyklus verwendet werden,

(8) Zuführen der Merkmalsvektoren einem Klassifikationsmodell, das für die jeweiligen Paare eine Klassifizierung in eine von mindestens zwei Klassen vornimmt, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören,

(9) Ausgeben von Informationen zumindest zu Paaren in der ersten Klasse.

Eine bevorzugte Ausführungsform des erfindungsgemäßen Systems umfasst:

- eine Eingabeeinheit,
- eine Steuer- und Recheneinheit,
- eine Merkmalsvektorerzeugungseinheit,
- eine Klassifikationseinheit, und
- eine Ausgabeeinheit,

wobei die Steuer- und Recheneinheit konfiguriert ist, über mehrere Biopanning-Zyklen, bei denen Antikörper und/oder Antikörperfragmente selektiert werden, Häufigkeiten von $V_L$-Genen, die variable Domänen von leichten Ketten der Antiköper und/oder Antikörperfragmente kodieren, und Häufigkeiten von $V_H$-Genen, die variable Domänen von schweren Ketten der Antiköper und/oder Antikörperfragmente kodieren, über die Eingabeeinheit zu erfassen,

wobei die Steuer- und Recheneinheit konfiguriert ist, die Merkmalsvektorerzeugungseinheit zu veranlassen Merkmalsvektoren für Paare der $V_L$- und $V_H$- Gene zu bilden, wobei als Merkmale die Häufigkeiten der $V_L$-Gene und der $V_H$-Gene eines Biopanning-Zyklus und eines vorangegangenen Biopanning-Zyklus verwendet werden,

wobei die Steuer- und Recheneinheit konfiguriert ist, die Klassifikationseinheit zu veranlassen, die Paare von $V_L$- und $V_H$-Genen anhand ihrer Merkmalsvektoren einer von mindestens zwei Klassen zuzuordnen, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören.

wobei die Steuer- und Recheneinheit konfiguriert ist, die Ausgabeeinheit zu veranlassen, zumindest Informationen über Paare auszugeben, die der ersten Klasse zugeordnet sind.

Eine bevorzugte Ausführungsform des erfindungsgemäßen Computerprogrammprodukts umfasst einen Datenträger, und Programmcode, der auf dem Datenträger gespeichert ist, und der einen Computer, in dessen Arbeitsspeicher der Programmcode geladen ist, dazu veranlasst, die folgenden Schritte auszuführen:

- Erfassen von Häufigkeiten von $V_L$-Genen, die variable Domänen von leichten Ketten von Antiköpern und/oder Antikörperfragmenten kodieren, und von Häufigkeiten von $V_H$-Genen, die variable Domänen von schweren Ketten der Antiköper und/oder Antikörperfragmente kodieren, über mehrere Zyklen eines Biopannings, in dem die Antiköper und/oder Antikörperfragmente selektiert werden,

- Bilden von Merkmalsvektoren für Paare der $V_L$- und $V_H$-Gene, wobei als Merkmale zumindest die Häufigkeiten der $V_L$-Gene und der $V_H$-Gene eines Biopanning-Zyklus und eines vorangegangenen Biopanning-Zyklus verwendet werden,

- Zuführen der Merkmalsvektoren einem Klassifikationsmodell, das Paare der $V_L$- und $V_H$-Gene auf Basis ihrer Merkmalsvektoren einer von mindestens zwei Klassen zuordnet, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören.

- Ausgeben von Informationen zumindest über Paare, die der ersten Klasse zugeordnet sind.

Die Erfindung wird nachstehend anhand von Beispielen und Figuren näher erläutert, ohne die Erfindung auf die in den Beispiel genannten oder in den Figuren gezeigten Merkmale und Merkmalskombinationen beschränken zu wollen.

Es zeigen:

Figur 1 zeigt ein Beispiel für eine Selektionshierarchie mit drei Ebenen. Figur 1 ist weiter oben näher beschrieben.

Figur 2 zeigt schematisch ein Beispiel für einen Biopanning-Zyklus.

In einem ersten Schritt wird eine Bibliothek (1) von Phagen (1a, 1b, 1c) bereitgestellt, bei denen Antikörper und/oder Antikörperfragmente (10, 11, 12) als Fusionspolypeptide auf Phagen-Hüllproteinen exprimiert sind.

Zusätzlich wird ein Substrat (2) mit immobilisierten Antigenen und/oder Antigenfragmenten bereitgestellt. Die immobilisierten Antigenen und/oder Antigenfragmenten weisen Bindungsstellen (2) auf, an denen die Antikörper und/oder Antikörperfragmente (10, 11, 12) der Phagen (1a, 1b, 1c) binden können.

In Schritt A des Biopanning-Zyklus werden die Phagen mit dem Substrat inkubiert.

Dabei kommt es zu Wechselwirkungen zwischen den Antikörpern/Antikörperfragmenten und den Antigenen. Der Antikörper/das Antikörperfragment (1c) passt im vorliegenden Fall genau zur Bindungstelle (20) des immobilisierten Antigens; die Wechselwirkung und die resultierende Bindung sind sehr stark.

In Schritt B des Biopanning-Zyklus werden die nicht oder nur schwach an das Substrat bindenden Phagen separiert (abgewaschen). Es verbleiben die stärker bindenden Phagen.

In Schritt C des Biopanning-Zyklus werden die stärker bindenden Phagen vom Substrat gelöst.

In Schritt D des Biopanning-Zyklus werden die vom Substrat gelösten Phagen vermehrt. Das Ergebnis ist eine neue Phagen-Bibliothek, die erneut einem Substrat ausgesetzt werden kann.

Von einem Teil der vom Substrat gelösten Phagen können die Gene der Antikörper und/oder Antikörperfragmente sequenziert werden (Schritt E). Dabei werden die $V_L$-Gene, die die variablen Domänen von leichten Ketten der Antikörper und/oder Antikörperfragmente kodieren und die $V_H$-Gene, die die variablen Domänen der schweren Ketten der Antikörper und/oder Antikörperfragmente kodieren, und vorzugsweise auch ihre Häufigkeiten ermittelt.

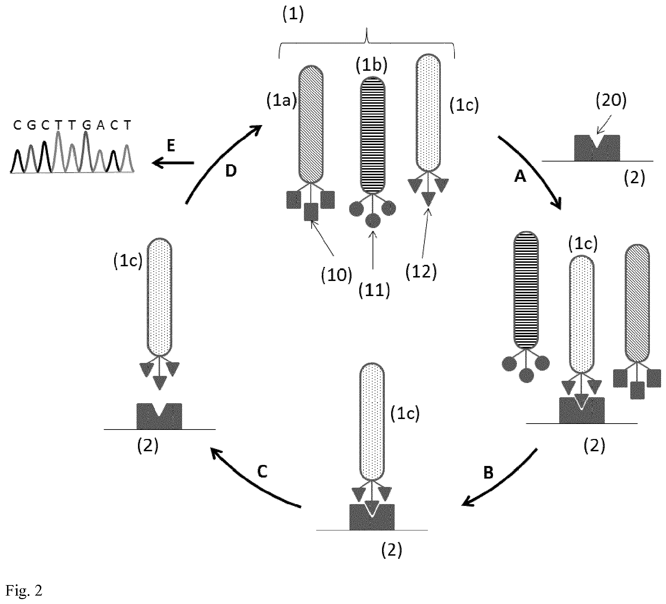

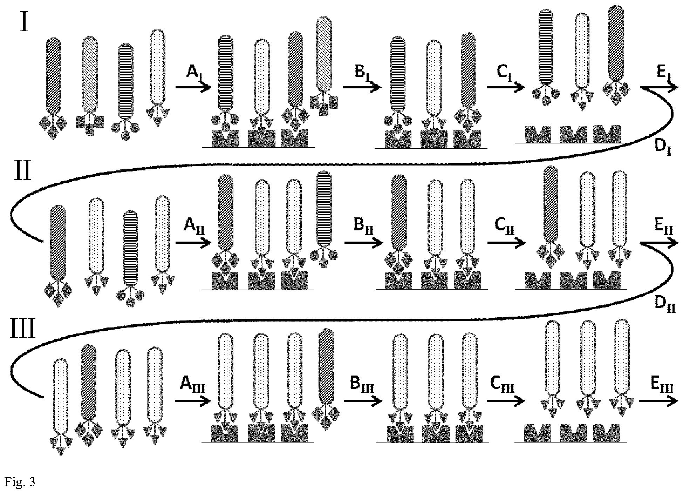

Figur 3 zeigt schematisch ein Biopanning mit drei Zyklen (I, II, III). Wird wie im vorliegenden Fall jeweils das gleiche Substrat eingesetzt, reichern sich die am stärksten bindenden Antikörper / Antikörperfragmente mit jedem Zyklus weiter an. Die Schritte $A_I$, $A_{II}$, $A_{III}$, $B_I$, $B_{II}$, $B_{III}$, $C_I$, $C_{II}$, $C_{III}$, $D_I$ $D_{II}$, $D_{III}$, $E_I$, $E_{II}$, und $E_{III}$ entsprechen den Schritten A, B, C, D und E in Figur 2.

Nach jedem Zyklus (I, II, II) können die $V_H$- und $V_L$-Gene und ihre Häufigkeiten ermittelt werden (Schritte $E_I$, $E_{II}$ und $E_{III}$).

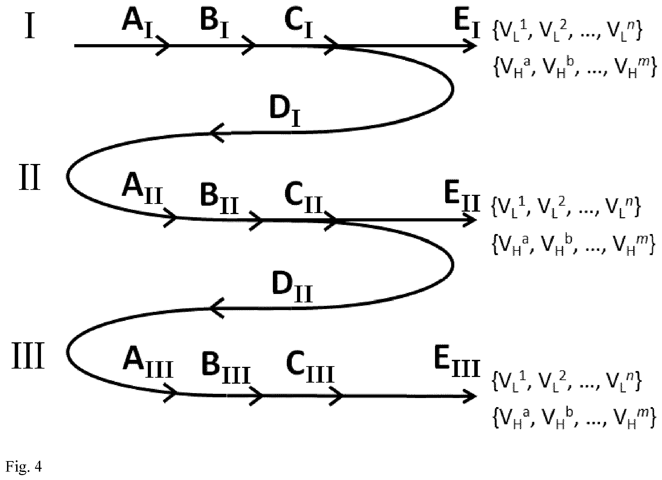

Figur 4 zeigt schematisch das gleiche Biopanning-Verfahren wie in Figur 3 mit den Zyklen I, II und II und den Schritten $A_I$, $A_{II}$, $A_{III}$, $B_I$, $B_{II}$, $B_{III}$, $C_I$, $C_{II}$, $C_{III}$, $D_I$, $D_{II}$, $D_{III}$, $E_I$, $E_{II}$ und $E_{III}$. Das Ergebnis der Schritte $E_I$, $E_{II}$ und $E_{III}$ ist eine Menge an $V_L$-Genen $\{V_L^1, V_L^2, ..., V_L^n\}$ und eine Menge an $V_H$-Genen $\{VH^a, VH^b, ..., VH^m\}$; jedes Gen ist anhand einer eindeutigen Kennung (1, 2, ... bis $n$ bzw. a, b, ... bis $m$) identifizierbar.

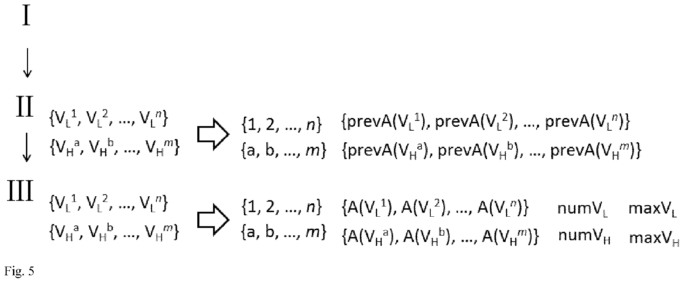

Figur 5 zeigt beispielhaft die Erzeugung/Erfassung von Merkmalen zu $V_L$-Genen und $V_H$-Genen. Es wurden drei Sequenzierungszyklen (I, II, II) durchlaufen. Im Anschluss an die Selektionszyklen II und III wurden die $V_L$-Gene und die $V_H$-Gene sequenziert.

Der Selektionszyklus II ist im vorliegenden Beispiel der "erste Selektionszyklus", der Selektionszyklus III ist der "zweite Selektionszyklus" im Sinne der vorliegenden Erfindung.

Merkmale zu den $V_L$-Genen und $V_H$-Genen sind beispielsweise die eindeutigen Kennungen ($\{1, 2, ..., n\}$ und $\{a, b, ..., m\}$).

Weitere Merkmale zu den $V_L$-Genen und $V_H$-Genen sind beispielsweise die Häufigkeiten der $V_L$-Gene im Pool aus dem zweiten Selektionszyklus $\{A(V_L^1), A(V_L^2), ..., A(V_L^n)\}$ und der $V_H$-Gene im Pool aus dem zweiten Selektionszyklus $\{A(V_H^a), A(V_H^b), ..., A(V_H^m)\}$.

Weitere Merkmale zu den $V_L$-Genen und $V_H$-Genen sind beispielsweise die Häufigkeiten der $V_L$-Gene im Pool aus dem ersten Selektionszyklus $\{prevA(V_L^1), prevA(V_L^2), ..., prevA(V_L^n)\}$ und der $V_H$-Gene im Pool aus dem ersten Selektionszyklus $\{prevA(V_H^a), prevA(V_H^b), ..., prevA(V_H^m)\}$.

Weitere Merkmale zu den $V_L$-Genen und $V_H$-Genen sind beispielsweise die absoluten Zahlen unterschiedlicher $V_L$-Gene ($numV_L$) und unterschiedlicher $V_H$-Gene ($numV_H$) in dem Pool aus dem zweiten Selektionszyklus.

Weitere Merkmale zu den $V_L$-Genen und $V_H$-Genen sind beispielsweise die absolute Zahl (Häufigkeit) desjenigen $V_H$-Gens, das in dem Pool aus dem zweiten Selektionszyklus am häufigsten vorhanden ist ($maxV_H$) und die absolute Zahl (Häufigkeit) desjenigen $V_L$-Gens, das in dem Pool aus dem zweiten Selektionszyklus am häufigsten vorhanden ist ($maxV_L$).

Weitere Merkmale lassen sich aus den beschriebenen Merkmalen errechnen, wie beispielsweise

$$relV_H = A(V_H)/maxV_H$$

$$relV_L = A(V_L)/maxV_L$$

$$diff = |A(V_H) - A(V_L)|$$

$$reldiff = |A(V_H) - A(V_L)| / Max(A(V_H), A(V_L))$$

$$prevdiff = |prevA(V_H) - prevA(V_L)|$$

$$prevRelDiffV_H = (|A(V_H) - prevA(V_H)|) / Max((A(V_H), prevA(V_H))$$

$$prevRelDiffV_L = (|A(V_L) - prevA(V_L)|) / Max((A(V_L), prevA(V_L))$$

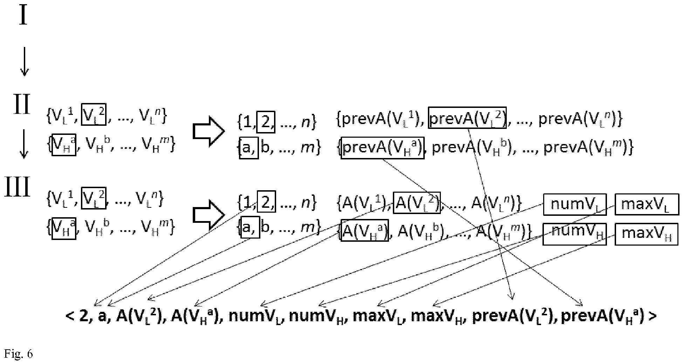

Die genannten Merkmale und/oder weitere/andere Merkmale werden zur Erzeugung von Merkmalsvektoren eingesetzt. Dies ist schematisch in Figur 6 dargestellt.

Figur 6 zeigt beispielhaft die Erzeugung eines Merkmalvektors für ein Paar von $V_L$-Genen und $V_H$-Genen.

Der Merkmalsvektor wird für das Paar $V_L^2$ und $V_H^a$ erzeugt.

Merkmale, die in den Merkmalsvektor einfließen sind:

- die Kennung des $V_L^2$-Gens: 2
- die Kennung des $V_H^a$-Gens: a
- die Häufigkeit des $V_L^2$-Gens im Pool aus dem zweiten Selektionszyklus: $A(V_L^2)$
- die Häufigkeit des $V_H^a$-Gens im Pool aus dem zweiten Selektionszyklus: $A(V_H^a)$
- die Häufigkeit des $V_L^2$-Gens im Pool aus dem ersten Selektionszyklus: $prevA(V_L^2)$
- die Häufigkeit des $V_H^a$-Gens im Pool aus dem ersten Selektionszyklus: $prevA(V_H^a)$
- die absolute Zahl unterschiedlicher $V_L$-Gene im Pool aus dem zweiten Selektionszyklus: $numV_L$
- die absolute Zahl unterschiedlicher $V_H$-Gene im Pool aus dem zweiten Selektionszyklus: $numV_H$
- die Häufigkeit desjenigen $V_H$-Gens, das in dem Pool aus dem zweiten Selektionszyklus am häufigsten vorhanden ist: $maxV_H$
- die Häufigkeit desjenigen $V_L$-Gens, das in dem Pool aus dem zweiten Selektionszyklus am häufigsten vorhanden ist: $maxV_L$.

Ein bevorzugter Merkmalsvektor für ein beliebiges Paar eines $V_L^n$-Gens und eines $V_H^m$-Gens mit den jeweiligen Kennungen $n$ und $m$ ist:

$<n, m, A(V_L^n), A(V_H^m), diff^{n,m}, relDiff^{n,m}, relV_H^m, relV_L^m, numV_H, numV_L, maxV_H, maxV_L, prevA(V_L^n), prevA(V_H^m), prevdiff^{n,m}, reldiffn,m, prevRelDifV_H^m, prevRelDiffV_L^m>$

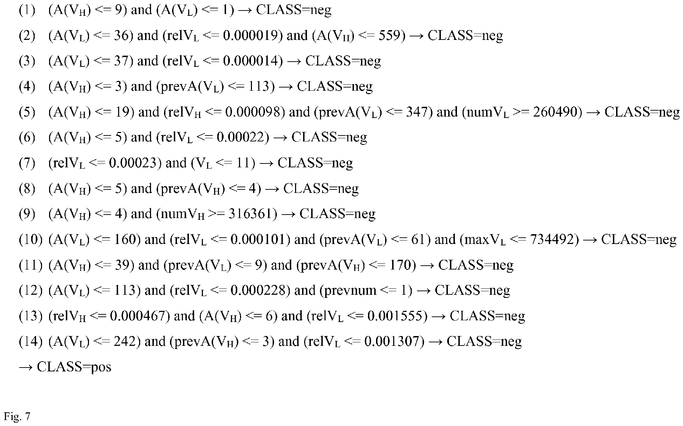

Figur 7 zeigt beispielhaft und schematisch einen Entscheidungsbaum, der ein Ergebnis der Erzeugung eines Klassifikationsmodells sein kann.

Es werden für ein Paar eines $V_L$-Gens und eines $V_H$-Gens nacheinander die Bedingungen (1) bis (14) geprüft. Trifft die Bedingungen (1) zu, dann handelt es sich um kein $V_L$-$V_H$-Paar (CLASS=neg). Die Klassifizierung ist abgeschlossen. Trifft Bedingung (1) nicht zu, wird Bedingung (2) geprüft. Trifft die Bedingung (2) zu, dann handelt es sich um kein $V_L$-$V_H$-Paar (CLASS=neg). Die Klassifizierung ist abgeschlossen. Trifft Bedingung (2) nicht zu, wird Bedingung (3) geprüft, und so fort.

Treffen die Bedingungen (1) bis (14) nicht zu, handelt es sich um ein $V_L$-$V_H$-Paar (CLASS=pos).

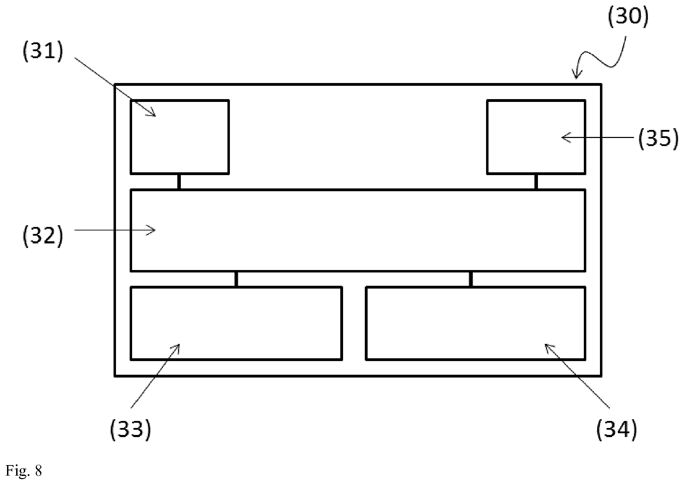

Figur 8 zeigt schematisch eine Ausführungsform des erfindungsgemäßen Systems.

Das System (30) umfasst eine Eingabeeinheit (31), eine Steuer- und Recheneinheit (32), eine Merkmalsvektorerzeugungseinheit (33), eine Klassifikationseinheit (34), und eine Ausgabeeinheit (35).

Über die Eingabeeinheit (31) werden Informationen in das System (30) eingebracht. Üblicherweise dient die Eingabeeinheit (31) auch als Kommunikationsschnittstelle mit einem Nutzer des Systems (30). Die Eingabeeinheit (31) kann eine Tastatur, eine Maus, einen Touchscreen, ein Mikrofon, ein Netzwerkverbindung, eine Verbindung zu einem Datenspeicher, ein Verbindung zu einem Gerät und/oder dergleichen umfassen. Insbesondere werden über die Eingabeeinheit (31) Merkmale zu $V_L$-Genen und $V_H$-Genen, die variable Domänen von leichten und schweren Ketten von Antiköpern und/oder Antikörperfragmenten kodieren, in das System eingebracht, um dort weiter verarbeitet zu werden. Dabei können diese Informationen manuell von einem Nutzer über eine Nutzerschnittstelle eingegeben und/oder automatisch über eine Schnittstelle aus einem Datenspeicher und/oder einem angeschlossenen und/oder über ein Netzwerk mit dem System (30) verbundenen Gerät (zum Beispiel einer Sequenzierungsvorrichtung) eingelesen werden. Es ist denkbar, dass über die Eingabeeinheit (31) nur einige der Merkmale zu $V_L$-Genen und $V_H$-Genen eingebracht werden, während andere Merkmale zu $V_L$-Genen und $V_H$-Genen durch die Steuer- und Recheneinheit (32) und/oder die Merkmalsvektorerzeugungseinheit (33) aus den eingebrachten Merkmalen berechnet werden.

Die Steuer- und Recheneinheit (32) dient der Steuerung der Komponenten des Systems (30) und der Koordinierung der Daten- und Signalflüsse zwischen den Komponenten und zwischen dem System und externen Geräten. Die Steuer- und Recheneinheit (32) umfasst üblicherweise einen Arbeitsspeicher, in den das erfindungsgemäße Computerprogramm geladen werden kann, um einen oder mehrere Schritte des erfindungsgemäßen Verfahrens auszuführen.

Die Merkmalsvektorerzeugungseinheit (33) erzeugt aus den Merkmalen zu den $V_L$-Genen und $V_H$-Genen für einzelne Paare von $V_L$-Genen und $V_H$-Genen Merkmalsvektoren. Die Merkmalsvektorerzeugungseinheit (33) kann ein Bestandteil der Steuer- und Recheneinheit (32) oder eine davon unabhängige Einheit sein. Es ist auch denkbar, dass Merkmalsvektoren über die Eingabeeinheit (31) in das System (30) eingebracht werden.

Die Klassifikationseinheit (34) führt eine Klassifizierung für Paare von $V_L$-Genen und $V_H$-Genen anhand des jeweiligen Merkmalsvektors durch. Für jedes Paar eines $V_L$-Gens und eines $V_H$-Gens existiert ein Merkmalsvektor, der der Klassifikationseinheit (34) zugeführt wird. Die Klassifikationseinheit (34) ordnet das jeweilige Paar anhand des jeweiligen Merkmalvektors einer von mindestens zwei Klassen zu, wobei eine erste Klasse Paare umfasst, bei denen das $V_L$-Gen und das $V_H$-Gen variable Domänen kodieren, die zum selben Antikörper und/oder Antikörperfragment gehören (CLASS=pos), und eine zweite Klasse Paare umfasst, bei denen das $V_L$-Gen und das $V_H$-Gen variable Domänen kodieren, die nicht zum selben Antikörper und/oder Antikörperfragment gehören (CLASS=neg). Die Klassifikationseinheit (34) kann ein Bestandteil der Steuer- und Recheneinheit (32) und/oder ein Bestandteil der Merkmalsvektorerzeugungseinheit (33) und/oder eine unabhängige Einheit sein.

Über die Ausgabeeinheit (35) wird das Ergebnis der Klassifizierung ausgegeben. Die Ausgabe erfolgt vorzugsweise gegenüber dem Nutzer des Systems (30). Die Ausgabeeinheit kann einen Bildschirm, einen Drucker, einen Lautsprecher, einen Datenspeicher, eine Verbindung zu einem Netzwerk, eine Verbindung zu einem Gerät und/oder dergleichen umfassen.

Das erfindungsgemäße System kann beispielsweise als ein Computer (z.B. Desktop-Computer, Tablet-Computer, Smartphone, Server) oder ein Verbund von Computern ausgestaltet sein.

**Patentansprüche**

1. Verfahren umfassend die Schritte:

   - Bereitstellen einer Bibliothek von Antikörpern und/oder Antikörperfragmenten
   - Zuführen der Antikörper und/oder Antikörperfragmente einem Selektionsverfahren, wobei das Selektionsverfahren mindestens zwei Selektionszyklen, einen ersten Selektionszyklus und einen zweiten Selektionszyklus, umfasst
   - Sequenzieren der Gene der Antikörper und/oder Antikörperfragmente nach dem ersten Selektionszyklus und nach dem zweiten Selektionszyklus und Ermitteln von $V_L$-Genen, die die variablen Domänen von leichten Ketten der Antikörper und/oder Antikörperfragmente kodieren und von $V_H$-Genen, die die variablen Domänen der schweren Ketten der Antikörper und/oder Antikörperfragmente kodieren

- Ermitteln von Merkmalen zu den $V_L$-Genen und $V_H$-Genen aus dem ersten Selektionsverfahren und aus dem zweiten Selektionsverfahren, wobei die Merkmale zu den $V_L$-Genen und $V_H$-Genen die folgenden Merkmale umfassen:

  • eindeutige Kennungen der ermittelten $V_H$-Gene,
  • eindeutige Kennungen der ermittelten $V_L$-Gene,
  • Häufigkeiten der $V_H$-Gene in dem Pool aus dem zweiten Selektionszyklus,
  • Häufigkeiten der $V_L$-Gene in dem Pool aus dem zweiten Selektionszyklus,
  • die Häufigkeiten der $V_H$-Gene in dem Pool aus dem ersten Selektionszyklus, und
  • die Häufigkeiten der $V_L$-Gene in dem Pool aus dem ersten Selektionszyklus

- Bilden von Merkmalsvektoren für Paare von $V_L$-Genen und $V_H$-Genen aus dem zweiten Selektionszyklus auf Basis der ermittelten Merkmale aus dem ersten Selektionszyklus und dem zweiten Selektionszyklus
- Zuführen der Merkmalsvektoren einem Klassifikationsmodell, das für die jeweiligen Paare eine Klassifizierung in eine von mindestens zwei Klassen vornimmt, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören
- Ausgeben von Informationen zumindest zu Paaren in der ersten Klasse.

2. Verfahren gemäß Anspruch 1, wobei Antikörper und/oder Antikörperfragmente, die aus dem ersten Selektionszyklus gewonnen werden, optional nach Durchführen eines Vermehrungsschrittes, in dem die gewonnenen Antikörper und/oder Antikörperfragmente vermehrt werden, dem zweiten Selektionszyklus zugeführt werden.

3. Verfahren gemäß einem der Ansprüche 1 oder 2, wobei es sich bei dem Selektionsverfahren um ein Biopanning-Verfahren handelt.

4. Verfahren gemäß einem der Ansprüche 1 bis 3, wobei das Sequenzieren mittels eines Next-Generation-Sequencing-Verfahrens erfolgt.

5. Verfahren gemäß einem der Ansprüche 1 bis 4, wobei eines oder mehrere weitere Merkmale zu den $V_L$-Genen und $V_H$-Genen aus der folgenden Liste ausgewählt sind:

  • die absolute Zahl unterschiedlicher $V_H$-Gene in dem Pool aus dem zweiten Selektionszyklus
  • die absolute Zahl unterschiedlicher $V_L$-Gene in dem Pool aus dem zweiten Selektionszyklus
  • die absolute Zahl desjenigen $V_H$-Gens, das in dem Pool aus dem zweiten Selektionszyklus am häufigsten vorhanden ist
  • die absolute Zahl desjenigen $V_L$-Gens, das in dem Pool aus dem zweiten Selektionszyklus am häufigsten vorhanden ist
  • die relative Häufigkeit der $V_H$-Gene in dem Pool aus dem zweiten Selektionszyklus bezogen auf die Zahl des am häufigsten in dem Pool aus dem zweiten Selektionszyklus auftretenden $V_H$-Gens
  • die relative Häufigkeit der $V_L$-Gene in dem Pool aus dem zweiten Selektionszyklus bezogen auf die Zahl des am häufigsten in dem Pool aus dem zweiten Selektionszyklus auftretenden $V_L$-Gens
  • die Unterschiede zwischen den Häufigkeiten der $V_H$-Gene in dem Pool aus dem zweiten Selektionszyklus und der $V_L$-Gene in dem Pool aus dem zweiten Selektionszyklus
  • die relativen Unterschiede zwischen den Häufigkeiten der $V_H$-Gene in dem Pool aus dem zweiten Selektionszyklus und den Häufigkeiten der $V_L$-Gene in dem Pool aus dem zweiten Selektionszyklus in Bezug auf die Häufigkeit des jeweiligen $V_H$-Gens oder $V_L$-Gens in dem Pool aus dem zweiten Selektionszyklus, das häufiger auftritt
  • die Zahl der Selektionszyklen, die vor dem zweiten Selektionszyklus durchlaufen worden sind
  • die Unterschiede zwischen den Häufigkeiten der $V_H$-Gene aus dem Pool des ersten Selektionszyklus und den Häufigkeiten der $V_L$-Gene aus dem Pool des ersten Selektionszyklus
  • die relativen Veränderungen der Zahlen der $V_H$-Gene vom ersten Selektionszyklus zum zweiten Selektionszyklus
  • die relativen Veränderungen der Zahlen der $V_L$-Gene vom ersten Selektionszyklus zum zweiten Selektionszyklus.

6. Verfahren gemäß einem der Ansprüche 1 bis 5, wobei das Klassifikationsmodell anhand von bekannten Antikörpern und/oder Antikörperfragmenten in einem überwachten Lernverfahren erstellt worden ist.

7. Verfahren gemäß einem der Ansprüche 1 bis 5, wobei das Klassifikationsmodell auf einem Random Forest oder Gradient Boosting Verfahren basiert.

8. Verfahren gemäß einem der Ansprüche 1 bis 7, umfassend die folgenden Schritte:

(1) Bereitstellen einer Bibliothek von Phagen, bei denen Antikörper und/oder Antikörperfragmente als Fusionspolypeptide auf Phagen-Hüllproteinen exprimiert sind,
(2) Inkubieren der Phagen mit einem Substrat,
(3) Separieren der an das Substrat bindenden Phagen von den nicht-bindenden Phagen,
(4) Sequenzieren der Gene der Antikörper und/oder Antikörperfragmente auf den bindenden oder nicht-bindenden Phagen und Ermitteln von $V_L$-Genen, die die variablen Domänen von leichten Ketten der Antikörper und/oder Antikörperfragmente kodieren und von $V_H$-Genen, die die variablen Domänen der schweren Ketten der Antikörper und/oder Antikörperfragmente kodieren
(5) Ermitteln der Häufigkeiten der $V_L$-Gene und der $V_H$-Gene,
(6) Vermehren der bindenden oder nicht bindenden Phagen und dabei Bilden einer neuen Bibliothek von Phagen und $n$-faches Wiederholen der Schritte (1) bis (5), wobei in Schritt (1) die jeweils neue Bibliothek von Phagen eingesetzt wird, wobei n eine ganze Zahl und größer als 0 ist, und wobei eine Durchführung der Schritte (1) bis (4) einen Biopanning-Zyklus darstellt,
(7) Bilden von Merkmalsvektoren für Paare von $V_L$-Genen und $V_H$-Genen, wobei als Merkmale zumindest die Häufigkeiten der $V_L$-Gene und der $V_H$-Gene des aktuellen Biopanning-Zyklus und eines vorangegangenen Biopanning-Zyklus verwendet werden,
(8) Zuführen der Merkmalsvektoren einem Klassifikationsmodell, das für die jeweiligen Paare eine Klassifizierung in eine von mindestens zwei Klassen vornimmt, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören,
(9) Ausgeben von Informationen zumindest zu Paaren in der ersten Klasse.

9. System umfassend:

- eine Eingabeeinheit,
- eine Steuer- und Recheneinheit,
- eine Merkmalsvektorerzeugungseinheit,
- eine Klassifikationseinheit, und
- eine Ausgabeeinheit,

- wobei die Steuer- und Recheneinheit konfiguriert ist, Merkmale zu $V_L$-Genen und $V_H$-Genen, die variable Domänen von leichten und schweren Ketten von Antiköpern und/oder Antikörperfragmenten kodieren, über die Eingabeeinheit zu erfassen,

o wobei die Antikörper und/oder Antikörperfragmente aus einem Selektionsverfahren umfassend mindestens zwei Selektionszyklen, einen ersten Selektionszyklus und einen zweiten Selektionszyklus, stammen,
o wobei die Merkmale zu den $V_L$-Genen und $V_H$-Genen die folgenden Merkmale umfassen:

• eindeutige Kennungen der ermittelten $V_H$-Gene,
• eindeutige Kennungen der ermittelten $V_L$-Gene,
• Häufigkeiten der $V_H$-Gene in dem Pool aus dem zweiten Selektionszyklus,
• Häufigkeiten der $V_L$-Gene in dem Pool aus dem zweiten Selektionszyklus,
• die Häufigkeiten der $V_H$-Gene in dem Pool aus dem ersten Selektionszyklus, und
• die Häufigkeiten der $V_L$-Gene in dem Pool aus dem ersten Selektionszyklus

- wobei die Steuer- und Recheneinheit konfiguriert ist, die Merkmalsvektorerzeugungseinheit zu veranlassen, auf Basis der erfassten Merkmale Merkmalsvektoren für Paare von $V_L$- und $V_H$- Genen aus dem zweiten Selektionszyklus zu bilden,

- wobei die Steuer- und Recheneinheit konfiguriert ist, die Klassifikationseinheit zu veranlassen, die Paare von $V_L$- und $V_H$-Genen anhand ihrer Merkmalsvektoren einer von mindestens zwei Klassen zuzuordnen, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören,

- wobei die Steuer- und Recheneinheit konfiguriert ist, die Ausgabeeinheit zu veranlassen, zumindest Informationen über Paare auszugeben, die der ersten Klasse zugeordnet sind.

10. System gemäß Anspruch 9, umfassend umfassend:

- eine Eingabeeinheit,
- eine Steuer- und Recheneinheit,
- eine Merkmalsvektorerzeugungseinheit,
- eine Klassifikationseinheit, und
- eine Ausgabeeinheit,

wobei die Steuer- und Recheneinheit konfiguriert ist, über mehrere Biopanning-Zyklen, bei denen Antikörper und/oder Antikörperfragmente selektiert werden, Häufigkeiten von $V_L$-Genen, die variable Domänen von leichten Ketten der Antiköper und/oder Antikörperfragmente kodieren, und Häufigkeiten von $V_H$-Genen, die variable Domänen von schweren Ketten der Antiköper und/oder Antikörperfragmente kodieren, über die Eingabeeinheit zu erfassen,

wobei die Steuer- und Recheneinheit konfiguriert ist, die Merkmalsvektorerzeugungseinheit zu veranlassen Merkmalsvektoren für Paare der $V_L$- und $V_H$- Gene zu bilden, wobei als Merkmale die Häufigkeiten der $V_L$-Gene und der $V_H$-Gene eines Biopanning-Zyklus und eines vorangegangenen Biopanning-Zyklus verwendet werden,

wobei die Steuer- und Recheneinheit konfiguriert ist, die Klassifikationseinheit zu veranlassen, die Paare von $V_L$- und $V_H$-Genen anhand ihrer Merkmalsvektoren einer von mindestens zwei Klassen zuzuordnen, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören.

wobei die Steuer- und Recheneinheit konfiguriert ist, die Ausgabeeinheit zu veranlassen, zumindest Informationen über Paare auszugeben, die der ersten Klasse zugeordnet sind.

11. Computerprogrammprodukt umfassend einen Datenträger, und Programmcode, der auf dem Datenträger gespeichert ist, und der einen Computer, in dessen Arbeitsspeicher der Programmcode geladen ist, dazu veranlasst, die folgenden Schritte auszuführen:

- Erfassen von Merkmalen zu $V_L$-Genen und $V_H$-Genen, die variable Domänen von leichten und schweren Ketten von Antiköpern und/oder Antikörperfragmenten kodieren,

  ∘ wobei die Antikörper und/oder Antikörperfragmente aus einem Selektionsverfahren umfassend mindestens zwei Selektionszyklen, einen ersten Selektionszyklus und einen zweiten Selektionszyklus, stammen,
  ∘ wobei die Merkmale zu den $V_L$-Genen und $V_H$-Genen die folgenden Merkmale umfassen:

    • eindeutige Kennungen der ermittelten $V_H$-Gene,
    • eindeutige Kennungen der ermittelten $V_L$-Gene,
    • Häufigkeiten der $V_H$-Gene in dem Pool aus dem zweiten Selektionszyklus,
    • Häufigkeiten der $V_L$-Gene in dem Pool aus dem zweiten Selektionszyklus,
    • die Häufigkeiten der $V_H$-Gene in dem Pool aus dem ersten Selektionszyklus, und
    • die Häufigkeiten der $V_L$-Gene in dem Pool aus dem ersten Selektionszyklus

- Bilden von Merkmalsvektoren für Paare von $V_L$- und $V_H$-Genen aus dem zweiten Selektionszyklus auf Basis der erfassten Merkmale,
- Zuordnen der Paare von $V_L$- und $V_H$-Genen auf Basis ihrer Merkmalsvektoren zu einer von mindestens zwei Klassen, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören,
- Ausgeben von Informationen zumindest über Paare, die der ersten Klasse zugeordnet sind.

12. Computerprogrammprodukt gemäß Anspruch 11 umfassend einen Datenträger, und Programmcode, der auf dem Datenträger gespeichert ist, und der einen Computer, in dessen Arbeitsspeicher der Programmcode geladen ist, dazu veranlasst, einen oder mehrere der Schritte eines Verfahrens gemäß einem der Ansprüche 1 bis 8 durchzuführen.

13. Computerprogrammprodukt gemäß einem der Ansprüche 11 bis 12, umfassend einen Datenträger, und Programmcode, der auf dem Datenträger gespeichert ist, und der einen Computer, in dessen Arbeitsspeicher der Programmcode geladen ist, dazu veranlasst, die folgenden Schritte auszuführen:

- Erfassen von Häufigkeiten von $V_L$-Genen, die variable Domänen von leichten Ketten von Antiköpern und/oder Antikörperfragmenten kodieren, und von Häufigkeiten von $V_H$-Genen, die variable Domänen von schweren Ketten der Antiköper und/oder Antikörperfragmente kodieren, über mehrere Zyklen eines Biopannings, in dem die Antiköper und/oder Antikörperfragmente selektiert werden,
- Bilden von Merkmalsvektoren für Paare der $V_L$- und $V_H$-Gene, wobei als Merkmale zumindest die Häufigkeiten der $V_L$-Gene und der $V_H$-Gene eines Biopanning-Zyklus und eines vorangegangenen Biopanning-Zyklus verwendet werden,
- Zuführen der Merkmalsvektoren einem Klassifikationsmodell, das Paare der $V_L$- und $V_H$-Gene auf Basis ihrer Merkmalsvektoren einer von mindestens zwei Klassen zuordnet, wobei eine erste Klasse Paare umfasst, bei denen das $V_L$-Gen und das $V_H$-Gen variable Domänen kodieren, die zum selben Antikörper und/oder Antikörperfragment gehören, und eine zweite Klasse Paare umfasst, bei denen das $V_L$-Gen und das $V_H$-Gen variable Domänen kodieren, die nicht zum selben Antikörper und/oder Antikörperfragment gehören.
- Ausgeben von Informationen zumindest über Paare, die der ersten Klasse zugeordnet sind.

**Claims**

1. Method comprising the steps of:

- providing a library of antibodies and/or antibody fragments
- introducing the antibodies and/or antibody fragments to a selection method, the selection method comprising at least two selection cycles, a first selection cycle and a second selection cycle
- sequencing the genes of the antibodies and/or antibody fragments after the first selection cycle and after the second selection cycle and ascertaining $V_L$ genes encoding the variable domains of light chains of the antibodies and/or antibody fragments and $V_H$ genes encoding the variable domains of the heavy chains of the antibodies and/or antibody fragments
- ascertaining features relating to the $V_L$ genes and $V_H$ genes from the first selection method and from the second selection method, the features relating to the $V_L$ genes and $V_H$ genes comprising the following features:

• unambiguous identifiers of the ascertained $V_H$ genes,
• unambiguous identifiers of the ascertained $V_L$ genes,
• counts of the $V_H$ genes in the pool from the second selection cycle,
• counts of the $V_L$ genes in the pool from the second selection cycle,
• the counts of the $V_H$ genes in the pool from the first selection cycle, and
• the counts of the $V_L$ genes in the pool from the first selection cycle

- forming feature vectors for pairs of $V_L$ genes and $V_H$ genes from the second selection cycle on the basis of the ascertained features from the first selection cycle and the second selection cycle
- introducing the feature vectors to a classification model which performs for the respective pairs a classification into one of at least two classes, a first class encompassing pairs in which the $V_L$ gene and the $V_H$ gene encode variable domains which belong to the same antibody and/or antibody fragment and a second class encompassing pairs in which the $V_L$ gene and the $V_H$ gene encode variable domains which do not belong to the same antibody and/or antibody fragment
- outputting information at least in relation to pairs in the first class.

2. Method according to Claim 1, wherein antibodies and/or antibody fragments obtained from the first selection cycle are introduced to the second selection cycle optionally after performance of a multiplication step in which the obtained antibodies and/or antibody fragments are multiplied.

3. Method according to either of Claims 1 and 2, wherein the selection method is a biopanning method.

4. Method according to any of Claims 1 to 3, wherein the sequencing is carried out by means of a next-generation sequencing method.

5. Method according to any of Claims 1 to 4, wherein one or more further features relating to the $V_L$ genes and $V_H$ genes are selected from the following list:

   • the absolute number of different $V_H$ genes in the pool from the second selection cycle
   • the absolute number of different $V_L$ genes in the pool from the second selection cycle
   • the absolute number of that $V_H$ gene which is present in the pool from the second selection cycle with the greatest count
   • the absolute number of that $V_L$ gene which is present in the pool from the second selection cycle with the greatest count
   • the relative count of the $V_H$ genes in the pool from the second selection cycle based on the number of the $V_H$ gene which occurs in the pool from the second selection cycle with the greatest count
   • the relative count of the $V_L$ genes in the pool from the second selection cycle based on the number of the $V_L$ gene which occurs in the pool from the second selection cycle with the greatest count
   • the differences between the counts of the $V_H$ genes in the pool from the second selection cycle and of the $V_L$ genes in the pool from the second selection cycle
   • the relative differences between the counts of the $V_H$ genes in the pool from the second selection cycle and the counts of the $V_L$ genes in the pool from the second selection cycle in relation to the count of the respective $V_H$ gene or $V_L$ gene in the pool from the second selection cycle that occurs with a greater count
   • the number of selection cycles which were passed through before the second selection cycle
   • the differences between the counts of the $V_H$ genes from the pool of the first selection cycle and the counts of the $V_L$ genes from the pool of the first selection cycle
   • the relative changes in the numbers of the $V_H$ genes from the first selection cycle to the second selection cycle
   • the relative changes in the numbers of the $V_L$ genes from the first selection cycle to the second selection cycle.

6. Method according to any of Claims 1 to 5, wherein the classification model has been created in a supervised learning method on the basis of known antibodies and/or antibody fragments.

7. Method according to any of Claims 1 to 5, wherein the classification model is based on a random forest or gradient boosting method.

8. Method according to any of Claims 1 to 7, comprising the following steps:

   (1) providing a library of phages in which antibodies and/or antibody fragments are expressed as fusion polypeptides on phage coat proteins,
   (2) incubating the phages with a substrate,
   (3) separating the substrate-binding phages from the nonbinding phages,
   (4) sequencing the genes of the antibodies and/or antibody fragments on the binding or nonbinding phages and ascertaining $V_L$ genes encoding the variable domains of light chains of the antibodies and/or antibody fragments and $V_H$ genes encoding the variable domains of the heavy chains of the antibodies and/or antibody fragments,
   (5) ascertaining the counts of the $V_L$ genes and the $V_H$ genes,
   (6) multiplying the binding or nonbinding phages and, in doing so, forming a new library of phages and repeating steps (1) to (5) n times, each new library of phages being used in step (1), *n* being an integer and greater than 0, and performance of steps (1) to (4) being one biopanning cycle,
   (7) forming feature vectors for pairs of $V_L$ genes and $V_H$ genes, the features used being at least the counts of the $V_L$ genes and the $V_H$ genes of the current biopanning cycle and of a preceding biopanning cycle,
   (8) introducing the feature vectors to a classification model which performs for the respective pairs a classification into one of at least two classes, a first class encompassing pairs in which the $V_L$ gene and the $V_H$ gene encode variable domains which belong to the same antibody and/or antibody fragment and a second class encompassing pairs in which the $V_L$ gene and the $V_H$ gene encode variable domains which do not belong to the same antibody and/or antibody fragment,

(9) outputting information at least in relation to pairs in the first class.

9. System comprising:

- an input unit,
- a control and calculation unit,
- a feature vector generation unit,
- a classification unit, and
- an output unit,

- the control and calculation unit being configured to acquire features relating to $V_L$ genes and $V_H$ genes encoding variable domains of light and heavy chains of antibodies and/or antibody fragments via the input unit,

∘ the antibodies and/or antibody fragments originating from a selection method comprising at least two selection cycles, a first selection cycle and a second selection cycle,
∘ the features relating to the $V_L$ genes and $V_H$ genes comprising the following features:

• unambiguous identifiers of the ascertained $V_H$ genes,
• unambiguous identifiers of the ascertained $V_L$ genes,
• counts of the $V_H$ genes in the pool from the second selection cycle,
• counts of the $V_L$ genes in the pool from the second selection cycle,
• the counts of the $V_H$ genes in the pool from the first selection cycle, and
• the counts of the $V_L$ genes in the pool from the first selection cycle

- the control and calculation unit being configured to prompt the feature vector generation unit to form feature vectors for pairs of $V_L$ and $V_H$ genes from the second selection cycle on the basis of the acquired features,
- the control and calculation unit being configured to prompt the classification unit to assign the pairs of $V_L$ and $V_H$ genes on the basis of their feature vectors to one of at least two classes, a first class encompassing pairs in which the $V_L$ gene and the $V_H$ gene encode variable domains which belong to the same antibody and/or antibody fragment and a second class encompassing pairs in which the $V_L$ gene and the $V_H$ gene encode variable domains which do not belong to the same antibody and/or antibody fragment,
- the control and calculation unit being configured to prompt the output unit to output at least information about pairs assigned to the first class.

10. System according to Claim 9, comprising comprising:

- an input unit,
- a control and calculation unit,
- a feature vector generation unit,
- a classification unit, and
- an output unit,

the control and calculation unit being configured to acquire, over multiple biopanning cycles in which antibodies and/or antibody fragments are selected, counts of $V_L$ genes encoding variable domains of light chains of the antibodies and/or antibody fragments and counts of $V_H$ genes encoding variable domains of heavy chains of the antibodies and/or antibody fragments via the input unit,
the control and calculation unit being configured to prompt the feature vector generation unit to form feature vectors for pairs of the $V_L$ and $V_H$ genes, the features used being the counts of the $V_L$ genes and the $V_H$ genes of a biopanning cycle and of a preceding biopanning cycle,
the control and calculation unit being configured to prompt the classification unit to assign the pairs of $V_L$ and $V_H$ genes on the basis of their feature vectors to one of at least two classes, a first class encompassing pairs in which the $V_L$ gene and the $V_H$ gene encode variable domains which belong to the same antibody and/or antibody fragment and a second class encompassing pairs in which the $V_L$ gene and the $V_H$ gene encode variable domains which do not belong to the same antibody and/or antibody fragment,
the control and calculation unit being configured to prompt the output unit to output at least information about pairs assigned to the first class.

11. Computer program product comprising a data carrier, and program code which is stored on the data carrier and which prompts a computer, in the memory of which the program code is loaded, to execute the following steps:

- acquiring features relating to $V_L$ genes and $V_H$ genes encoding variable domains of light and heavy chains of antibodies and/or antibody fragments,

  o the antibodies and/or antibody fragments originating from a selection method comprising at least two selection cycles, a first selection cycle and a second selection cycle,
  o the features relating to the $V_L$ genes and $V_H$ genes comprising the following features:

  • unambiguous identifiers of the ascertained $V_H$ genes,
  • unambiguous identifiers of the ascertained $V_L$ genes,
  • counts of the $V_H$ genes in the pool from the second selection cycle,
  • counts of the $V_L$ genes in the pool from the second selection cycle,
  • the counts of the $V_H$ genes in the pool from the first selection cycle, and
  • the counts of the $V_L$ genes in the pool from the first selection cycle

- forming feature vectors for pairs of $V_L$ and $V_H$ genes from the second selection cycle on the basis of the acquired features,
- assigning the pairs of $V_L$ and $V_H$ genes on the basis of their feature vectors to one of at least two classes, a first class encompassing pairs in which the $V_L$ gene and the $V_H$ gene encode variable domains which belong to the same antibody and/or antibody fragment and a second class encompassing pairs in which the $V_L$ gene and the $V_H$ gene encode variable domains which do not belong to the same antibody and/or antibody fragment,
- outputting information at least about pairs assigned to the first class.

12. Computer program product according to Claim 11, comprising a data carrier, and program code which is stored on the data carrier and which prompts a computer, in the memory of which the program code is loaded, to carry out one or more of the steps of a method according to any of Claims 1 to 8.

13. Computer program product according to any of Claims 11 to 12, comprising a data carrier, and program code which is stored on the data carrier and which prompts a computer, in the memory of which the program code is loaded, to execute the following steps:

- acquiring counts of $V_L$ genes encoding variable domains of light chains of antibodies and/or antibody fragments and counts of $V_H$ genes encoding variable domains of heavy chains of the antibodies and/or antibody fragments over multiple cycles of a biopanning procedure in which the antibodies and/or antibody fragments are selected,
- forming feature vectors for pairs of the $V_L$ and $V_H$ genes, the features used being at least the counts of the $V_L$ genes and the $V_H$ genes of a biopanning cycle and of a preceding biopanning cycle,
- introducing the feature vectors to a classification model which assigns pairs of the $V_L$ and $V_H$ genes on the basis of their feature vectors to one of at least two classes, a first class encompassing pairs in which the $V_L$ gene and the $V_H$ gene encode variable domains which belong to the same antibody and/or antibody fragment and a second class encompassing pairs in which the $V_L$ gene and the $V_H$ gene encode variable domains which do not belong to the same antibody and/or antibody fragment,
- outputting information at least about pairs assigned to the first class.

**Revendications**

1. Procédé comprenant les étapes suivantes :

- la préparation d'une bibliothèque d'anticorps et/ou de fragments d'anticorps,
- l'introduction des anticorps et/ou des fragments d'anticorps dans un procédé de sélection, le procédé de sélection comprenant au moins deux cycles de sélection, un premier cycle de sélection et un deuxième cycle de sélection,
- le séquençage des gènes des anticorps et/ou des fragments d'anticorps après le premier cycle de sélection et après le deuxième cycle de sélection et la détermination de gènes $V_L$, qui codent les domaines variables de chaînes légères des anticorps et/ou des fragments d'anticorps, et de gènes $V_H$, qui codent les domaines variables des chaînes lourdes des anticorps et/ou des fragments d'anticorps,

- la détermination de caractéristiques relatives aux gènes $V_L$ et aux gènes $V_H$ du premier procédé de sélection et du deuxième procédé de sélection, les caractéristiques relatives aux gènes $V_L$ et aux gènes $V_H$ comprenant les caractéristiques suivantes :

- des identificateurs explicites des gènes $V_H$ déterminés,
- des identificateurs explicites des gènes $V_L$ déterminés,
- des fréquences des gènes $V_H$ dans le pool du deuxième cycle de sélection,
- des fréquences des gènes $V_L$ dans le pool du deuxième cycle de sélection,
- les fréquences des gènes $V_H$ dans le pool du premier cycle de sélection, et
- les fréquences des gènes $V_L$ dans le pool du premier cycle de sélection,

- la formation de vecteurs de caractéristiques pour des paires de gènes $V_L$ et de gènes $V_H$ issus du deuxième cycle de sélection sur la base des caractéristiques déterminées à partir du premier cycle de sélection et du deuxième cycle de sélection,
- l'introduction des vecteurs de caractéristiques dans un modèle de classification, qui réalise pour les paires respectives une classification dans une parmi au moins deux classes, une première classe comprenant des paires dans lesquelles le gène $V_L$ et le gène $V_H$ codent des domaines variables qui appartiennent au même anticorps et/ou fragment d'anticorps, et une deuxième classe comprenant des paires dans lesquelles le gène $V_L$ et le gène $V_H$ codent des domaines variables qui n'appartiennent pas au même anticorps et/ou fragment d'anticorps,
- la sortie d'informations relatives au moins à des paires dans la première classe.

2. Procédé selon la revendication 1, dans lequel des anticorps et/ou des fragments d'anticorps qui ont été obtenus à partir du premier cycle de sélection sont introduits dans le deuxième cycle de sélection, éventuellement après la réalisation d'une étape de multiplication, dans laquelle les anticorps et/ou fragments d'anticorps obtenus sont multipliés.

3. Procédé selon l'une quelconque des revendications 1 et 2, le procédé de sélection consistant en un procédé de biopannage.

4. Procédé selon l'une quelconque des revendications 1 à 3, dans lequel le séquençage a lieu au moyen d'un procédé de séquençage de nouvelle génération.

5. Procédé selon l'une quelconque des revendications 1 à 4, dans lequel une ou plusieurs caractéristiques supplémentaires relatives aux gènes $V_L$ et aux gènes $V_H$ sont choisies dans la liste suivante :

- le nombre absolu de gènes $V_H$ différents dans le pool du deuxième cycle de sélection,
- le nombre absolu de gènes $V_L$ différents dans le pool du deuxième cycle de sélection,
- le nombre absolu du gène $V_H$ qui est présent le plus fréquemment dans le pool du deuxième cycle de sélection,
- le nombre absolu du gène $V_L$ qui est présent le plus fréquemment dans le pool du deuxième cycle de sélection,
- la fréquence relative des gènes $V_H$ dans le pool du deuxième cycle de sélection par rapport au nombre du gène $V_H$ apparaissant le plus fréquemment dans le pool du deuxième cycle de sélection,
- la fréquence relative des gènes $V_L$ dans le pool du deuxième cycle de sélection par rapport au nombre du gène $V_L$ apparaissant le plus fréquemment dans le pool du deuxième cycle de sélection,
- les différences entre les fréquences des gènes $V_H$ dans le pool du deuxième cycle de sélection et des gènes $V_L$ dans le pool du deuxième cycle de sélection,
- les différences relatives entre les fréquences des gènes $V_H$ dans le pool du deuxième cycle de sélection et les fréquences des gènes $V_L$ dans le pool du deuxième cycle de sélection, au regard de la fréquence du gène $V_H$ ou du gène $V_L$ respectif dans le pool du deuxième cycle de sélection, qui apparaît plus fréquemment,
- le nombre de cycles de sélection qui se sont déroulés avant le deuxième cycle de sélection,
- les différences entre les fréquences des gènes $V_H$ du pool du premier cycle de sélection et les fréquences des gènes $V_L$ du pool du premier cycle de sélection,
- les modifications relatives des nombres des gènes $V_H$ du premier cycle de sélection au deuxième cycle de sélection,
- les modifications relatives des nombres des gènes $V_L$ du premier cycle de sélection au deuxième cycle de sélection.

6. Procédé selon l'une quelconque des revendications 1 à 5, dans lequel le modèle de classification a été établi à partir d'anticorps et/ou de fragments d'anticorps connus dans un procédé d'apprentissage supervisé.

7. Procédé selon l'une quelconque des revendications 1 à 5, dans lequel le modèle de classification est fondé sur un procédé Random Forest ou Gradient Boosting.

8. Procédé selon l'une quelconque des revendications 1 à 7, comprenant les étapes suivantes :

(1) la préparation d'une bibliothèque de phages, dans lesquels des anticorps et/ou des fragments d'anticorps sont exprimés en tant que polypeptides de fusion sur des protéines d'enveloppe de phages,
(2) l'incubation des phages avec un substrat,
(3) la séparation des phages reliés au substrat d'avec les phages non reliés,
(4) le séquençage des gènes des anticorps et/ou des fragments d'anticorps sur les phages reliés ou non reliés et la détermination de gènes $V_L$, qui codent les domaines variables de chaînes légères des anticorps et/ou des fragments d'anticorps, et de gènes $V_H$, qui codent les domaines variables des chaînes lourdes des anticorps et/ou des fragments d'anticorps,
(5) la détermination des fréquences des gènes $V_L$ et des gènes $V_H$,
(6) la multiplication des phages reliés ou non reliés et ainsi la formation d'une nouvelle bibliothèque de phages et la répétition n fois des étapes (1) à (5), la nouvelle bibliothèque respective de phages étant utilisée dans l'étape (1), n étant un nombre entier et supérieur à 0, et une réalisation des étapes (1) à (4) constituant un cycle de biopannage,
(7) la formation de vecteurs de caractéristiques pour des paires de gènes $V_L$ et de gènes $V_H$, au moins les fréquences des gènes $V_L$ et des gènes $V_H$ du cycle de biopannage actuel et d'un cycle de biopannage précédent étant utilisées en tant que caractéristiques,
(8) l'introduction des vecteurs de caractéristiques dans un modèle de classification, qui réalise pour les paires respectives une classification dans une parmi au moins deux classes, une première classe comprenant des paires dans lesquelles le gène $V_L$ et le gène $V_H$ codent des domaines variables qui appartiennent au même anticorps et/ou fragment d'anticorps, et une deuxième classe comprenant des paires dans lesquelles le gène $V_L$ et le gène $V_H$ codent des domaines variables qui n'appartiennent pas au même anticorps et/ou fragment d'anticorps,
(9) la sortie d'informations relatives au moins à des paires dans la première classe.

9. Système comprenant :

- une unité d'entrée,
- une unité de commande et de calcul,
- une unité de génération de vecteurs de caractéristiques,
- une unité de classification, et
- une unité de sortie,

  - l'unité de commande et de calcul étant configurée pour acquérir des caractéristiques relatives à des gènes $V_L$ et à des gènes $V_H$, qui codent des domaines variables de chaînes légères et lourdes d'anticorps et/ou de fragments d'anticorps, par l'intermédiaire de l'unité d'entrée,

    ○ les anticorps et/ou les fragments d'anticorps provenant d'un procédé de sélection qui comprend au moins deux cycles de sélection, un premier cycle de sélection et un deuxième cycle de sélection,
    ○ les caractéristiques relatives aux gènes $V_L$ et aux gènes $V_H$ comprenant les caractéristiques suivantes :

      • des identificateurs explicites des gènes $V_H$ déterminés,
      • des identificateurs explicites des gènes $V_L$ déterminés,
      • des fréquences des gènes $V_H$ dans le pool du deuxième cycle de sélection,
      • des fréquences des gènes $V_L$ dans le pool du deuxième cycle de sélection,
      • les fréquences des gènes $V_H$ dans le pool du premier cycle de sélection, et
      • les fréquences des gènes $V_L$ dans le pool du premier cycle de sélection,

  - l'unité de commande et de calcul étant configurée pour demander à l'unité de génération de vecteurs de caractéristiques de former des vecteurs de caractéristiques pour des paires de gènes $V_L$ et $V_H$ du deuxième cycle de sélection sur la base des caractéristiques acquises,
  - l'unité de commande et de calcul étant configurée pour demander à l'unité de classification d'associer les paires de gènes $V_L$ et $V_H$ à partir de leurs vecteurs de caractéristiques à une parmi au moins deux classes, une première classe comprenant des paires dans lesquelles le gène $V_L$ et le gène $V_H$ codent des domaines variables qui appartiennent au même anticorps et/ou fragment d'anticorps, et une deuxième classe comprenant des paires dans lesquelles le gène $V_L$ et le gène $V_H$ codent des domaines variables qui n'appartiennent pas au même anticorps et/ou fragment d'anticorps,

- l'unité de commande et de calcul étant configurée pour demander à l'unité de sortie de sortir au moins des informations sur des paires qui sont associées à la première classe.

**10.** Système selon la revendication 9, comprenant comprenant :

- une unité d'entrée,
- une unité de commande et de calcul,
- une unité de génération de vecteurs de caractéristiques,
- une unité de classification, et
- une unité de sortie,

l'unité de commande et de calcul étant configurée pour acquérir, par l'intermédiaire de plusieurs cycles de biopannage, dans lesquels des anticorps et/ou des fragments d'anticorps sont sélectionnés, des fréquences de gènes $V_L$, qui codent des domaines variables de chaînes légères d'anticorps et/ou de fragments d'anticorps, et des fréquences de gènes $V_H$, qui codent des domaines variables de chaînes lourdes d'anticorps et/ou de fragments d'anticorps, par l'intermédiaire de l'unité d'entrée,

l'unité de commande et de calcul étant configurée pour demander à l'unité de génération de vecteurs de caractéristiques de former des vecteurs de caractéristiques pour des paires des gènes $V_L$ et $V_H$, les fréquences des gènes $V_L$ et des gènes $V_H$ d'un cycle de biopannage et d'un cycle de biopannage précédent étant utilisées en tant que caractéristiques,

l'unité de commande et de calcul étant configurée pour demander à l'unité de classification d'associer les paires de gènes $V_L$ et $V_H$ à partir de leurs vecteurs de caractéristiques à une parmi au moins deux classes, une première classe comprenant des paires dans lesquelles le gène $V_L$ et le gène $V_H$ codent des domaines variables qui appartiennent au même anticorps et/ou fragment d'anticorps, et une deuxième classe comprenant des paires dans lesquelles le gène $V_L$ et le gène $V_H$ codent des domaines variables qui n'appartiennent pas au même anticorps et/ou fragment d'anticorps,

- l'unité de commande et de calcul étant configurée pour demander à l'unité de sortie de sortir au moins des informations sur des paires qui sont associées à la première classe.

**11.** Produit programme informatique comprenant un support de données et un code de programme, qui est stocké sur le support de données, et qui demande à un ordinateur, dans la mémoire de travail duquel le code de programme est chargé, de réaliser les étapes suivantes :

- l'acquisition de caractéristiques relatives à des gènes $V_L$ et des gènes $V_H$, qui codent des domaines variables de chaînes légères et lourdes d'anticorps et/ou de fragments d'anticorps,

  ◦ les anticorps et/ou les fragments d'anticorps provenant d'un procédé de sélection qui comprend au moins deux cycles de sélection, un premier cycle de sélection et un deuxième cycle de sélection,
  ◦ les caractéristiques relatives aux gènes $V_L$ et aux gènes $V_H$ comprenant les caractéristiques suivantes :

    • des identificateurs explicites des gènes $V_H$ déterminés,
    • des identificateurs explicites des gènes $V_L$ déterminés,
    • des fréquences des gènes $V_H$ dans le pool du deuxième cycle de sélection,
    • des fréquences des gènes $V_L$ dans le pool du deuxième cycle de sélection,
    • les fréquences des gènes $V_H$ dans le pool du premier cycle de sélection, et
    • les fréquences des gènes $V_L$ dans le pool du premier cycle de sélection,

- la formation de vecteurs de caractéristiques pour des paires de gènes $V_L$ et de gènes $V_H$ issus du deuxième cycle de sélection sur la base des caractéristiques acquises,
- l'association des paires de gènes $V_L$ et $V_H$ sur la base de leurs vecteurs de caractéristiques à une parmi au moins deux classes, une première classe comprenant des paires dans lesquelles le gène $V_L$ et le gène $V_H$

codent des domaines variables qui appartiennent au même anticorps et/ou fragment d'anticorps, et une deuxième classe comprenant des paires dans lesquelles le gène $V_L$ et le gène $V_H$ codent des domaines variables qui n'appartiennent pas au même anticorps et/ou fragment d'anticorps,
- la sortie d'informations au moins sur des paires qui sont associées à la première classe.

12. Produit programme informatique selon la revendication 11, comprenant un support de données et un code de programme, qui est stocké sur le support de données, et qui demande à un ordinateur, dans la mémoire de travail duquel le code de programme est chargé, de réaliser une ou plusieurs des étapes d'un procédé selon l'une quelconque des revendications 1 à 8.

13. Produit programme informatique selon l'une quelconque des revendications 11 et 12, comprenant un support de données et un code de programme, qui est stocké sur le support de données, et qui demande à un ordinateur, dans la mémoire de travail duquel le code de programme est chargé, de réaliser les étapes suivantes :

- l'acquisition de fréquences de gènes $V_L$, qui codent des domaines variables de chaînes légères d'anticorps et/ou de fragments d'anticorps, et de fréquences de gènes $V_H$, qui codent des domaines variables de chaînes lourdes des anticorps et/ou des fragments d'anticorps, par l'intermédiaire de plusieurs cycles d'un biopannage, dans lequel les anticorps et/ou les fragments d'anticorps sont sélectionnés,
- la formation de vecteurs de caractéristiques pour des paires des gènes $V_L$ et $V_H$, au moins les fréquences des gènes $V_L$ et des gènes $V_H$ d'un cycle de biopannage et d'un cycle de biopannage précédent étant utilisées en tant que caractéristiques,
- l'introduction des vecteurs de caractéristiques dans un modèle de classification, qui associe des paires des gènes $V_L$ et $V_H$ sur la base de leurs vecteurs de caractéristiques à une parmi au moins deux classes, une première classe comprenant des paires dans lesquelles le gène $V_L$ et le gène $V_H$ codent des domaines variables qui appartiennent au même anticorps et/ou fragment d'anticorps, et une deuxième classe comprenant des paires dans lesquelles le gène $V_L$ et le gène $V_H$ codent des domaines variables qui n'appartiennent pas au même anticorps et/ou fragment d'anticorps,
- la sortie d'informations au moins sur des paires qui sont associées à la première classe.

Fig. 1

Fig. 2

Fig. 3

Fig. 4

I

↓

II $\{V_L^1, V_L^2, ..., V_L^n\}$ $\Rightarrow$ $\{1, 2, ..., n\}$ $\{\text{prevA}(V_L^1), \text{prevA}(V_L^2), ..., \text{prevA}(V_L^n)\}$

↓ $\{V_H^a, V_H^b, ..., V_H^m\}$ $\{a, b, ..., m\}$ $\{\text{prevA}(V_H^a), \text{prevA}(V_H^b), ..., \text{prevA}(V_H^m)\}$

III $\{V_L^1, V_L^2, ..., V_L^n\}$ $\Rightarrow$ $\{1, 2, ..., n\}$ $\{A(V_L^1), A(V_L^2), ..., A(V_L^n)\}$ $\text{numV}_L$ $\text{maxV}_L$

$\{V_H^a, V_H^b, ..., V_H^m\}$ $\{a, b, ..., m\}$ $\{A(V_H^a), A(V_H^b), ..., A(V_H^m)\}$ $\text{numV}_H$ $\text{maxV}_H$

Fig. 5

Fig. 6

(1)  $(A(V_H) <= 9)$ and $(A(V_L) <= 1) \rightarrow$ CLASS=neg

(2)  $(A(V_L) <= 36)$ and $(relV_L <= 0.000019)$ and $(A(V_H) <= 559) \rightarrow$ CLASS=neg

(3)  $(A(V_L) <= 37)$ and $(relV_L <= 0.000014) \rightarrow$ CLASS=neg

(4)  $(A(V_H) <= 3)$ and $(prevA(V_L) <= 113) \rightarrow$ CLASS=neg

(5)  $(A(V_H) <= 19)$ and $(relV_H <= 0.000098)$ and $(prevA(V_L) <= 347)$ and $(numV_L >= 260490) \rightarrow$ CLASS=neg

(6)  $(A(V_H) <= 5)$ and $(relV_L <= 0.00022) \rightarrow$ CLASS=neg

(7)  $(relV_L <= 0.00023)$ and $(V_L <= 11) \rightarrow$ CLASS=neg

(8)  $(A(V_H) <= 5)$ and $(prevA(V_H) <= 4) \rightarrow$ CLASS=neg

(9)  $(A(V_H) <= 4)$ and $(numV_H >= 316361) \rightarrow$ CLASS=neg

(10) $(A(V_L) <= 160)$ and $(relV_L <= 0.000101)$ and $(prevA(V_L) <= 61)$ and $(maxV_L <= 734492) \rightarrow$ CLASS=neg

(11) $(A(V_H) <= 39)$ and $(prevA(V_L) <= 9)$ and $(prevA(V_H) <= 170) \rightarrow$ CLASS=neg

(12) $(A(V_L) <= 113)$ and $(relV_L <= 0.000228)$ and $(prevnum <= 1) \rightarrow$ CLASS=neg

(13) $(relV_H <= 0.000467)$ and $(A(V_H) <= 6)$ and $(relV_L <= 0.001555) \rightarrow$ CLASS=neg

(14) $(A(V_L) <= 242)$ and $(prevA(V_H) <= 3)$ and $(relV_L <= 0.001307) \rightarrow$ CLASS=neg

$\rightarrow$ CLASS=pos

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2013178370 A1

**In der Beschreibung aufgeführte Nicht-Patentliteratur**

- **SAI T. REDDY et al.** Monoclonal antibodies isolated without screening by anaylzing the variable-gene repertoire of plasma cells. *Nature Biotechnology,* September 2010, vol. 28 (9), 965-971
- Hidden Lineage Complexity of Glycan-Dependent HIV-1 Broadly Neutralizing Antibodies Uncovered by Digital Panning and Native-Like gp140 Trimer. *Frontiers in Immunology,* 24. August 2017, vol. 8
- **SAI T. REDDY et al.** Monoclonal antibodies isolated without screening by anaylzing the variable-gene repertoire ofplasma cells. *Nature Biotechnology,* September 2010, vol. 28 (9), 965-971
- *Nature Biotechnology,* September 2010, vol. 28 (9), 965-971